# EUROPEAN PATENT SPECIFICATION

(11) **EP 2 876 441 B1**
(45) Date of publication and mention of the grant of the patent: **25.10.2017**
(21) Application number: 13194536.2
(22) Date of filing: 26.11.2013
(51) Int. Cl.: G01N 33/50, G01N 21/64

(54) **Quantitative analysis of contact-depending cell-to-cell transfer and disease transmission**
Quantitative Analyse von kontaktabhängigem Transfer von Zelle zur Zelle und Krankheitsübertragung
Analyse quantitative de transfert intercellulaire en fonction du contact et transmission de maladies

(43) Date of publication of application: 27.05.2015
(73) Proprietor: Bergen Teknologioverføring AS, 5006 Bergen (NO)
(72) Inventor: Kögel, Tanja, 5099 Bergen (NO); Hodneland, Erlend, 5099 Bergen (NO); Gerdes, Hans-Hermann, deceased (NO); Frei, Dominik Michael, 5008 Bergen (NO); Burtey, Anne, 5003 Bergen (NO)
(74) Representative: Benedum, Ulrich Max

(56) References cited:
- EP-A2- 2 058 658
- WO-A1-2006/125674
- BUKORESHTLIEV N V ET AL: "Selective block of tunneling nanotube (TNT) formation inhibits intercellular organelle transfer between PC12 cells", FEBS LETTERS, ELSEVIER, AMSTERDAM, NL, vol. 583, no. 9, 6 May 2009 (2009-05-06), pages 1481-1488, XP026085218, ISSN: 0014-5793, DOI: 10.1016/J.FEBSLET.2009.03.065 [retrieved on 2009-04-02]
- ELLIOTT G ET AL: "INTERCELLULAR TRAFFICKING AND PROTEIN DELIVERY BY A HERPESVIRUS STRUCTURAL PROTEIN", CELL, CELL PRESS, US, vol. 88, 24 January 1997 (1997-01-24), pages 223-233, XP000961185, ISSN: 0092-8674, DOI: 10.1016/S0092-8674(00)81843-7
- HODNELAND ERLEND ET AL: "Automated detection of tunneling nanotubes in 3D images", CYTOMETRY. PART A, JOHN WILEY, HOBOKEN, NJ, US, vol. 69A, no. 9, 1 September 2006 (2006-09-01), pages 961-972, XP009114263, ISSN: 1552-4922, DOI: 10.1002/CYTO.A.20302
- DIERK WITTIG ET AL: "Multi-Level Communication of Human Retinal Pigment Epithelial Cells via Tunneling Nanotubes", PLOS ONE, vol. 7, no. 3, E33195, 22 March 2012 (2012-03-22), pages 1-12, XP055099753, DOI: 10.1371/journal.pone.0033195
- RAINY N ET AL: "H-Ras transfers from B to T cells via tunneling nanotubes.", CELL DEATH & DISEASE 2013, vol. 4, E726, 2013, pages 1-9, XP002719584, ISSN: 2041-4889

## Description

### FIELD OF THE INVENTION

The invention relates to a method for quantitative analysis of transport processes between cells.

### BACKGROUND OF THE INVENTION

Molecular communication and substance transfer between mammalian cells can take place through a variety of pathways. The paracrine and endocrine signalling pathways for example require exocytosis into extracellular fluids or blood circulation followed by an uptake of the released substance by other cells expressing the respective receptors. The intercellular transport of materials by extracellular fluids does not require cells to be in physical contact to each other. For communication and material transfer through direct contact, the mammalian organisms provide gap- and tight- and adhesive junctions.

Tunnelling nanotubes (hereinafter TNTs) have been described as a transient form of contact-dependent cell-to-cell communication and biological material transfer (Rustom et al., Science 2004,303(5660):1007-10). TNTs have been shown to provide a structural basis for cell-to-cell communication in most cells, not only for cells *in vitro* but also for several tissues *in vivo* (Chinnery et. al, J. Immunol. 2008, 180(9):577983; Gurke et al, Histochem Cell Biol. 2008, 129:539-550; Gerdes et al., Mech Dev. 2013, 130(6-8):381-7). Moreover, a transfer of biological material and even an exchange of organelles may take place along *de novo* formed TNTs (Rustom et al., Science. 2004, 303(5660):1007-10; Onfelt et al., J. Immunol 2006, 173(3):1511-1513; Gerdes et al., Mech Dev. 2013,130(6-8):381-7). Thus, *de novo* formed TNTs are considered playing a role in the intercellular transmission of pathogens and diseases (Gousset et al., Nat. Cell Biol. 2008 11(3):328-36, Mukerji et al, Retrovirology 2012, 9:33ff; Eugenin et al, Cell Immunol. 2009; 254(2):142-148; Sowinski et al, Methods 2010,53:27-33; Rechavi et al, Genes Dev. 200923:1971-1979; Bishai et al, Cellular Microbiology 2013, 15:353-367; Review by Ireton K in Open Biol. 2013 3,130079; Fukumatsu et al, Cell Host & Microbe 2012, 11:325-336; Paloheimo et al, J Virol 2011, 85(13)6714-6724; Lamers et al, Current HIV research 2010,8(8):638; Costanzo et al, J Cell Sci. 2013, 126(16):367885;. Abela et al., PLoS Pathog. 2012, 8(4):e1002634; Monel at al., J Virol. 2012, 86(7):3924-33).

The quantitative analysis of contact-dependent cell-to-cell transfer however has proven very difficult, if not impossible, as *de novo* formed TNTs between mammalian cells are very delicate structures and easily disrupted. Furthermore it is difficult to create homogeneous conditions with equal spacing between cells in order to visualize and count TNTs. Conventional methods for quantitative analysis of intercellular transfer are typically based on differently labelled donor and acceptor cells which are plated and co-cultured on a substrate and subsequently analyzed, for example by flow cytometric analysis (Bukoreshtliev et al., FEBS Lett. 2009, 6;583(9):1481-8; EP 1 454 136B1, see example 14). The conventional methods used so far for quantitative analysis have numerous disadvantages. Above all, flow cytometric analysis demands a disruption of the spatial organization of the cultured cells so that a number of biologically interesting parameters are lost. The flow cytometric methods can further not reliably differentiate between contact-dependent cell-to-cell substance transfers and other intercellular transport mechanisms based on exocytosis, endocytosis and phagocytosis. Phagocytosis of stained cell debris is in particular a predominant process when the density of 1:1 co-cultured cells on a substrate is approaching confluency. Analysis by flow cytometry therefore provides merely data on the number of acceptor cells having newly incorporated or attached a detectable signal from donor cells. The usual test with conditioned medium has several flaws. For example it does not take into account degradation of the transferred substance during the incubation period. Also, during the medium conditioning phase, some of the transferred material will be taken up by the cells and not be available for the transfer test. Therefore it cannot be quantitative. Also in a Transwell® chamber it cannot be excluded that material that normally is transferred through the medium is not passing the membrane. The distance the particles need to travel may be too long or the mesh not permissive enough. Therefore, there is no good method of examining a test substance for its quantitative effect on contact-dependent cell-to-cell molecular communication, material transfer, and/or disease transmission. The prior art therefore represents a problem.

### SUMMARY OF THE INVENTION

The problem is solved by the method of claim 1. Further aspects of the invention and preferred embodiments thereof have been disclosed in the dependent claims.

The instant disclosure provides a microscope-based method for quantitative analysis of contact-dependent cell-to-cell molecular communication and transfer between cells, the method comprising the steps:
(i) obtaining of first singularized cells containing or expressing one or more traceable substances to provide donor cells;
(ii) obtaining of second singularized cells not containing or expressing said one or more traceable substances to provide acceptor cells;
(iii) combining said donor and acceptor cells in a ratio so that a majority of donor cells are surrounded by acceptor cells when grown in co-culture on a substrate and that the donor cells are essentially more than 200 micrometers apart from each other to obtain neighborhood regions wherein a contact-dependent cell-to-cell substance transfer can take place between said one donor cell and its surrounding acceptor cells,
(iv) culturing of said donor cells and acceptor cells in a culture medium in the presence of a substrate for a first period of time to allow donor and acceptor cells to attach to said substrate;
(v) replacing said culture medium by a medium wherein the proliferation of cells is inhibited and optionally washing cells attached to said substrate in order to remove cell debris;
(vi) co-culturing of said donor and acceptor cells in said proliferation inhibiting medium for a second period of time to allow for contact-dependent cell-to-cell transfer between said donor and acceptor cells;
(vii) imaging the donor and acceptor cells for microscopy to identify single donor cells which are more than 200 micrometers apart from the next donor cell but in contact to surrounding acceptor cells and defining a region of interest (ROI) containing in a space volume the identified single donor cell and a number of surrounding acceptor cells in contact relationship;
(viii) determining the amounts of traceable substance contained within the volume picture elements (volumetric pixel or voxel) of said region of interest (ROI) and defining the volume picture elements which are located within the cell boundaries of said donor and the number of acceptor cells by cell segmentation; and
(ix) calculating the amount of traceable substance present within the volume elements located within the one or more acceptor cells surrounding the single donor cell to determine the amount of contact-dependent cell-to-cell substance transfer between said donor cells and surrounding acceptor cells. This method can be used for a reliable quantitative analysis of contact-dependent cell-to-cell molecular communication and/or substance transfer between said donor and acceptor cells when examining a statistically significant number of single donor cells and surrounding acceptor cells. While described for a multiple donor cells, the method can also be applied on pairs of one donor cell and one acceptor cell.

It is further preferred to determine a background value of traceable substance or staining contained in volume elements of the region of interest (ROI) which are located within representative cells that have not received any traceable substance by contact-dependent cell-to-cell substance transfer. These cells are identified by a distance of 200 micrometers or more from any donor cell. The latter value corresponds to the background caused by medium-mediated intercellular transport such as diffusion, exocytosis, endocytosis, phagocytosis, pinocytosis or other vesicular transport of materials and cell debris, including that released by apoptotic or disrupted cells. If the measured transfers within those cell volumes increases in an experimental condition, this has its reason in the up-regulation of a medium-mediated form of transfer.

The imaging and/or staining of the donor and acceptor cells may be done after cell fixation and by conventional surface staining of the plasma membranes. Alternatively, live imaging of the cells may be used to determine the location and volume of donor and acceptor cells within the region of interest in time lapse experiments. The donor and acceptor cells may be of the same type or different, and optionally a third type of cells may be added as helper cell.

Another aspect relates to a method wherein a test substance is present or added to the culture medium prior the analysis of contact-dependent cell-to-cell transfer. The test substance may be a chemical compound or a pharmaceutically active agent, tool or treatment suspected having activity on the contact-dependent cell-to-cell transfer. Thus, the present disclosure encompasses a screening of pharmaceutically active agents to fight cell-to-cell transmission of viral, bacterial and parasitic diseases, and in the context of cancer development, notably of cancer cell to CAF (cancer associated fibroblasts) interactions.

In its broadest sense the method may also comprise a testing of physical treatments such as the application of heat or electromagnetic waves. The method may also be used for a screening of active compounds for treatment of cancer, tumors, metabolism disorders, nervous system disorders, circulatory disorders etc., as well as for testing substances and compositions for their effects in gene therapy, cell targeting and pharmacology.

Alternatively, the added agent or substance may have a known activity on cell-to-cell molecular communication so that the types of contact-dependent cell-to-cell transport processes can be examined further, for example, with respect to a *de novo* formation of TNTs or formed bicellular and tricellular gap, adherens- or tight junctions.

A main advantage of the disclosed method over flow cytometric analysis lies therein that it allows a clear differentiation between transfers wherein the traceable substance is located within the volumes defined by the plasma membranes of the cells, and transfers wherein the traceable substance adhere to the external plasma membrane. Another advantage is that the cell-to-cell substance transfer can be quantified with high accuracy. An accurate quantification of the cell-to-cell substance transfer which can be correlated to the donor cell by a microscope-based method has not been contemplated by the prior art and no methods were available for a screening of the effects of a test substance or agent on contact-dependent cell-to-cell transfer and molecular communication. Thus, the method can be used for quantitative analysis of the contact-dependent cell-to-cell transmission of a disease or infectious agent and/or a targeted active agent for pharmacokinetic studies.

introduction of a non-adhesive gap within the culture - where cells cannot adhere - provides a test/proof of the contact-dependent nature of the transfer for each new chosen transfer marker when analyzing the transfer that crosses the gap compared to the transfer extending to a longer distance than the gap width in other directions.

Further advantages, features and embodiments are disclosed in the detailed description and in the examples. It is understood, however, that the desired scope of protection is not limited to the embodiments described but defined in the claims. The invention is best understood, however, when read in conjunction with the accompanying figures which serve to illustrate preferred embodiments.

### BRIEF DESCRIPTION OF THE FIGURES

In the figures and drawings appended hereto:
- Figs. 1 A-A': are micrographs of fixed HeLa-Kyoto cells covering a region of interest (ROI) comprising one donor cell and surrounding acceptor cells (donor/acceptor cell ratio = 1:400) and of the intercellular substance transfers as represented by the DiD fluorescent signal, while the plasma membrane of the cells has been stained using AF488-wheat germ agglutinin;
- Fig. 1 A"-A"': are micrographs of fixed HeLa-Kyoto cells as shown in Figs 1 A-A' but covering a region of interest (field of view) with no donor cells;
- Figs. 2 A-B: are microscope images of live HeLa-Kyoto cells covering a region of interest (ROI) comprising one donor cell and surrounding acceptor cells (donor/acceptor cell ratio = 1:400) cultured in the presence (A) and absence (B) of low melting point agarose wherein the filtered images on the right show the intercellular substance transfers by the DiD fluorescent signal;
- Fig.2 C: is a diagram with a quantitative analysis of the average number of cells positive for DiD signal, comparing the presence and absence of low melting agarose and effect on intercellular substance transfers;
- Fig.3A: is a micrograph of HeLa-Kyoto cells stained grown on two adhesive stripes separated by one stripe with a non-adhesive BSA-coating as barrier (staining with celltracker™ green;
- Fig 3 B: is a micrograph of the Hela-Kyoto cells of Fig. 3A filtered for DiD, showing that insignificant amounts of substance transfer occurred across the nonadhesive barrier;
- Figs. 4A-B": are representative images of the cell segmentation process performed manually (A-A") and automatically (B-B") as disclosed by Hodneland et al in Source Code Biol Med. 2013, 8(1):16;
- Figs. 5 A-B: are bar diagrams comparing the quantified DiD transfer upon manual and automated mark staining;
- Figs. 6 A-A": are representative microscopic images of the DiD fluorescent signal in case of low (A), medium (A') and high (A") cell-to-cell substance transfer;
- Fig. 6 B-D: are plots with various quantitative analyses of the integrated DiD transfer intensity of the same set of data;
- Fig. 7 A: is bar diagram comparing DiD transfers in cultures with different number of cells per culture dish as determined by flow cytometry;
- Figs. 7 B-C: are representative microscopic images of the DiD-transfer in HeLa-Kyoto cells plated without (B) and in the presence (C) of low-melting point agarose;

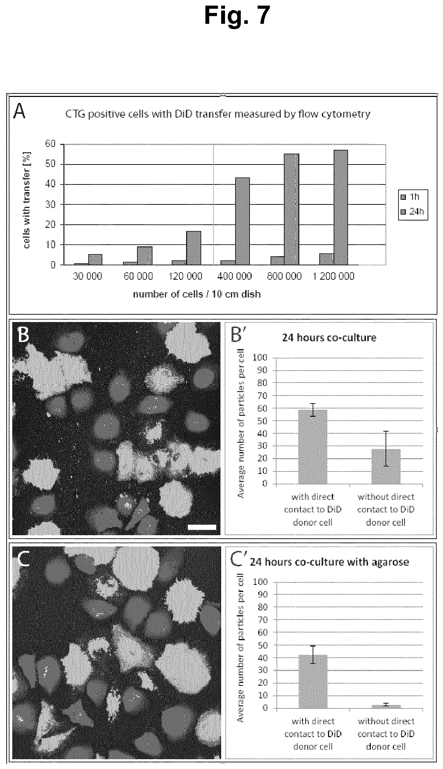

- Figs. 7 B'-C': are bar diagrams with quantitative analyses of the DiD transfer without (B') or in the presence (C') of low-melting point agarose as in Figs. 7B-C;

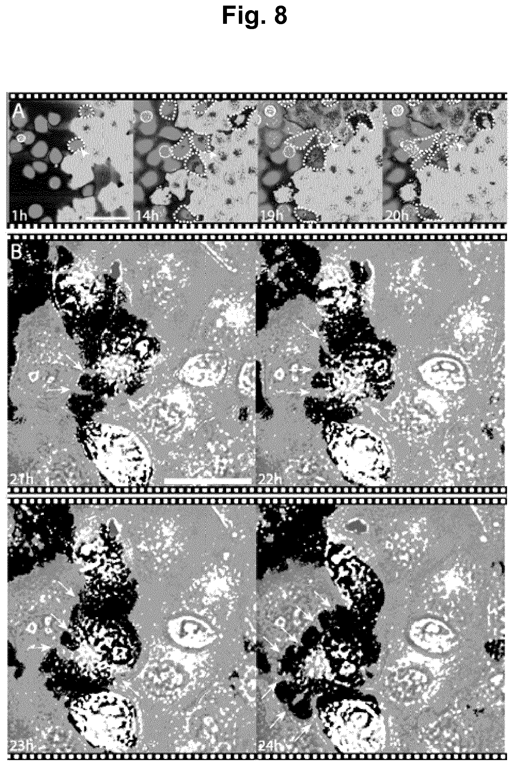

- Figs. 8 A-B: are micrographs showing a cell-to-cell transfer of vesicles in living DiD- and CTG-stained cells (A) and the corresponding dynamic interplay (B) of the vesicles with the protrusion bundles during cell division and at the end of mitosis;

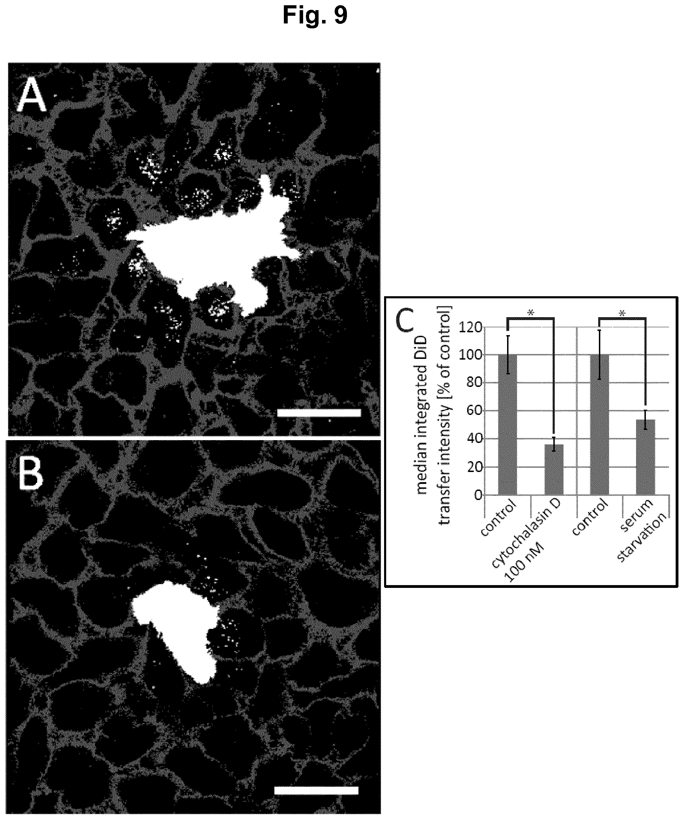

- Figs. 9 A-B: are representative micrographs of the DiD transfer in HeLa-Kyoto cells for a control (A) and under cytochalasin-D (B) conditions;
- Fig. 9 C: is a bar diagram with an automated quantitative analysis of cell-to-cell DiD organelle transfer in A-B and under serum starvation;

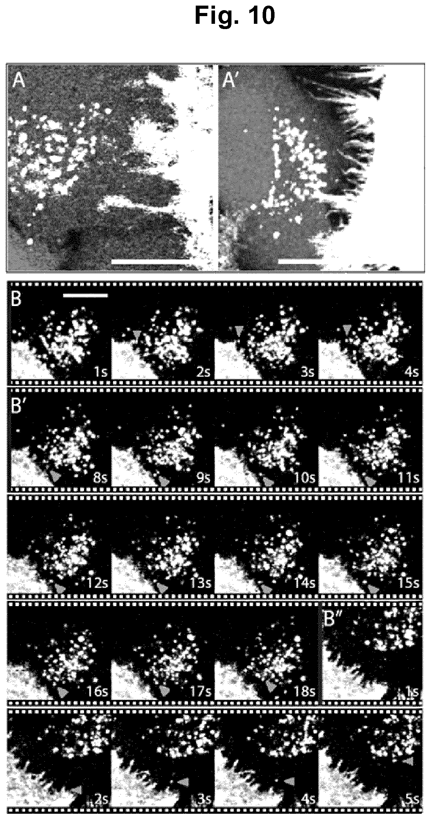

- Figs. 10 A-B": are representative micrographs of the morphology of cell-cell contact sites in HeLa-Kyoto cells at different planes (A and A') and time lapse imaging of the transfer process (B-B", arrowheads);

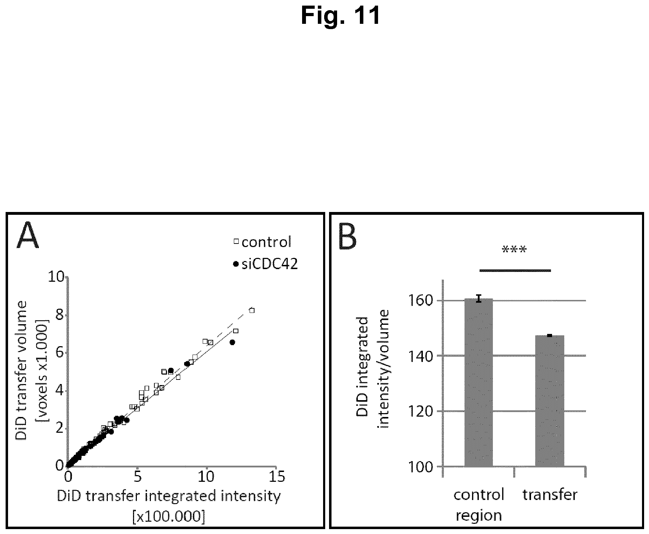

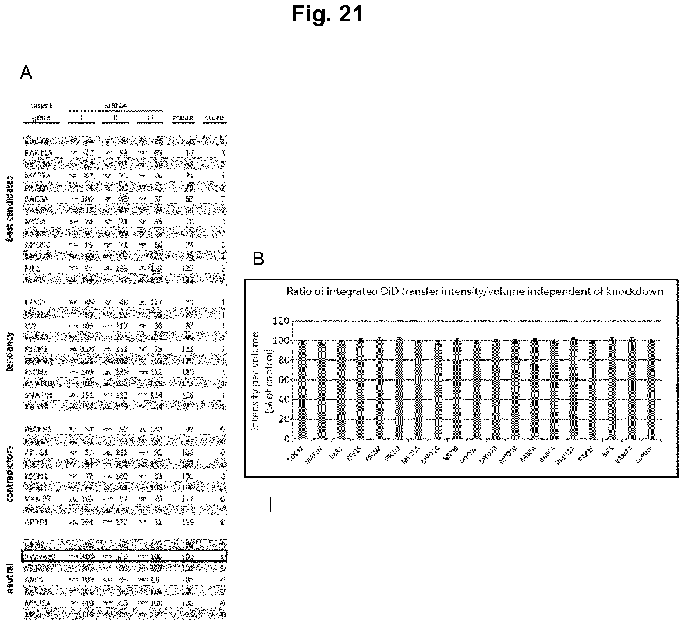

- Figs. 11 A: s a plot of the DiD integrated fluorescence intensity against the volume of transferred fluorescence for a control co-culture (A, open dots) and a coculture n which donor cells were treated with siRNA against CDC42, as an example (A. black dots), depicting no significant different density of the substance transfer regions upon CDC42 knockdown even though the amount of transfer significantly decreased (see Fig 21);
- Fig. 11B: is a bar diagram comparing the DiD integrated intensity per volume for a ROI containing no donor cell (representing contact-independent transfer) control and a substance transfer region, depicting significantly different density of contact-dependent versus contact independent transfer;

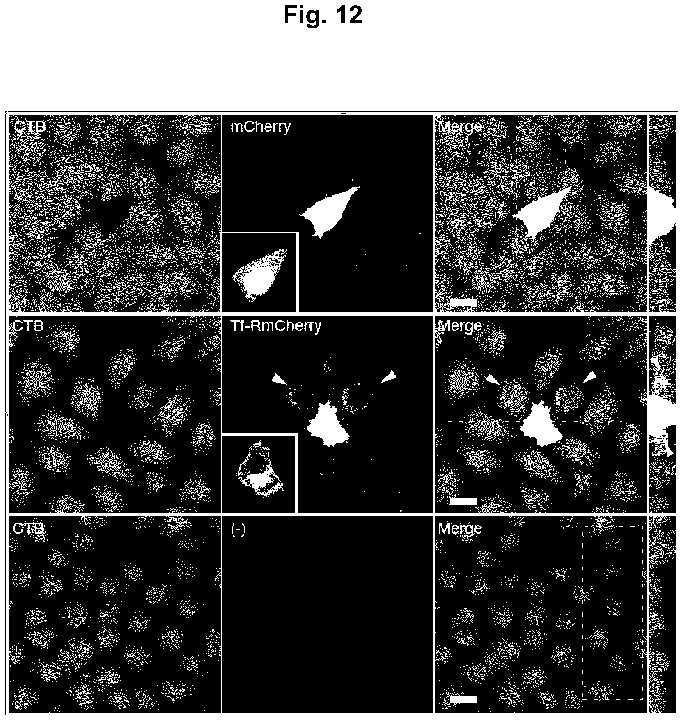

- Fig. 12: shows representative micrographs of substance transfers in HeLa-Kyoto cells of mCherry-labelled transferrin receptor (middle panel, TfRmCherry);mCherry alone (middle panel, mCherry) and within control regions without donor cells (middle panel, (-));

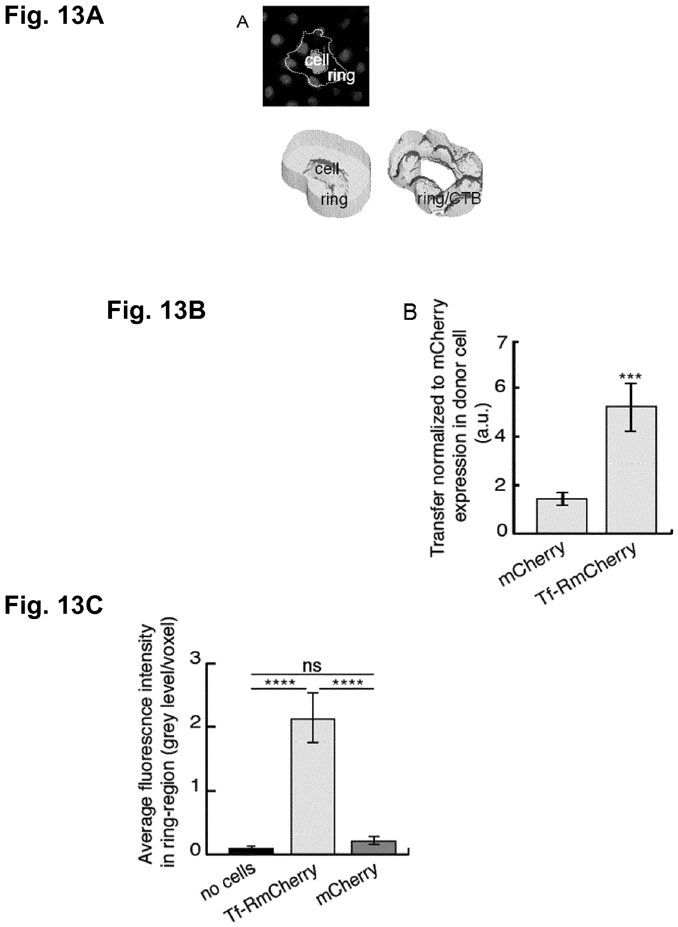

- Fig. 13 A: shows an example of the synthetic regions corresponding to the first ring of acceptor cells around donor cells, created automatically instead of/as an alternative to cell segmentation if cells are not membrane-stained;
- Figs. 13 B-C: are bar diagrams comparing the transfer of the transferrin receptor mCherry or mCherry alone (C) or normalized to the level of expression of mcherry proteins in donor cells (B) and analyzed by automated quantification of fluorescence in ring regions;

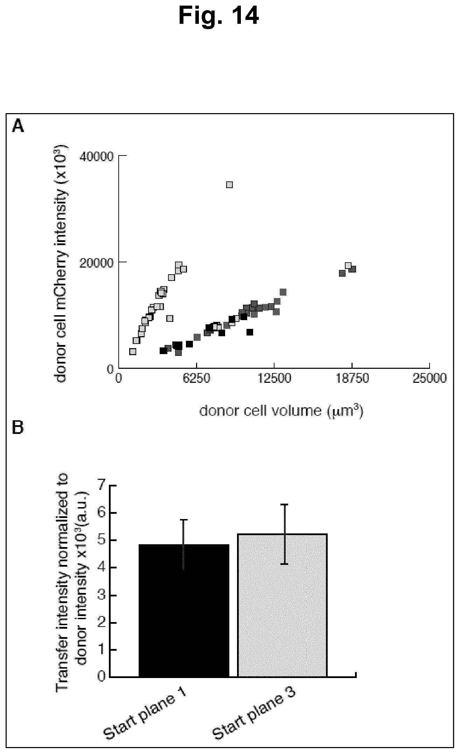

- Fig.14 A: is a plot showing mCherry expression against the volume of donor cells;
- Fig.14 B: are bar diagrams comparing normalized transferrin receptor mcherry transfer intensity measured over the entire range (start plane 1) or excluding the first two micrometers above substrate (start plane 3);

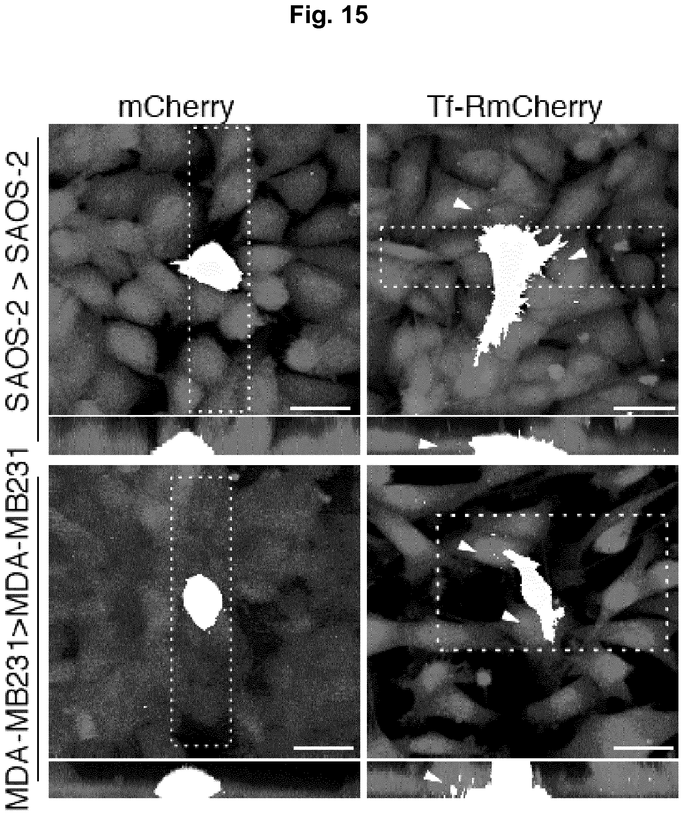

- Fig. 15: are micrographs showing the transfer of mCherry-labelled transferrin receptor (Tf-RmCherry) and mCherry alone in homotypic co-cultures ofosteosarcoma (SAOS-2) and breast cancer cells (MDA-MB231);

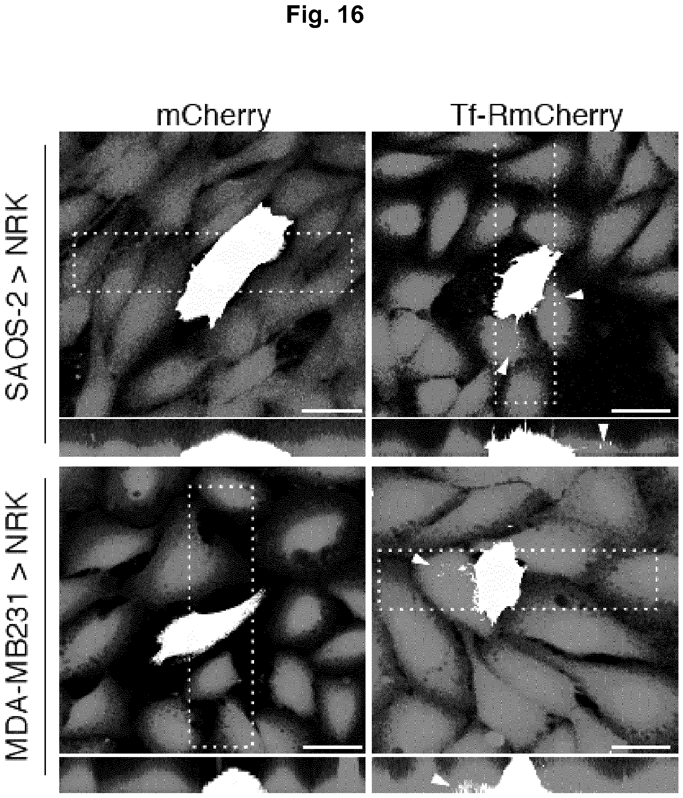

- Fig. 16: are micrographs showing the transfer of transferrin receptor (TfRmCherry) or mCherry alone in heterotypic co-cultures from donor cancer cells to fibroblastic NRK cells;

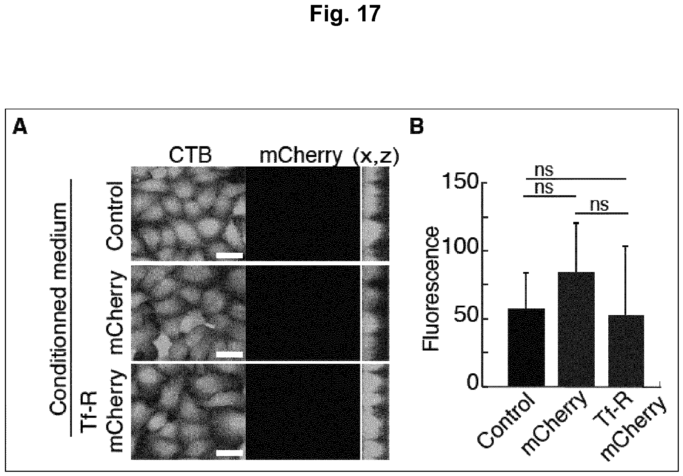

- Fig. 17 A: are micrographs showing mCherry fluorescence (non-present) in acceptor cells cultured without donor cells but using conditioned medium;
- Fig. 17 B: is a bar diagram with a comparison of mCherry fluorescence as in Fig.17A based on a quantification of fluorescence;

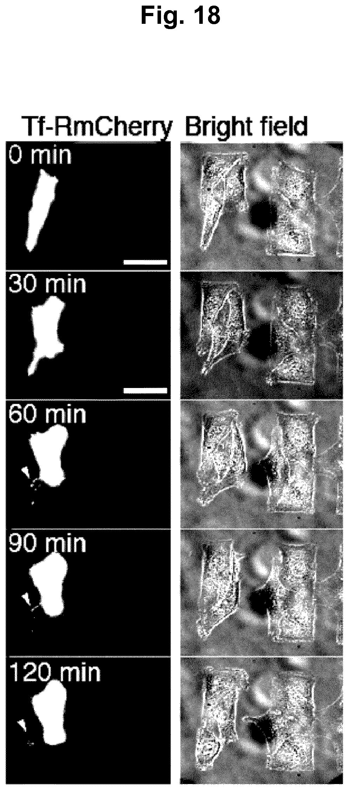

- Fig. 18: are time lapse micrographs showing a cell-to-cell transfer of mCherry transferrin in HeLa-Kyoto cells on micro-patterned surfaces;

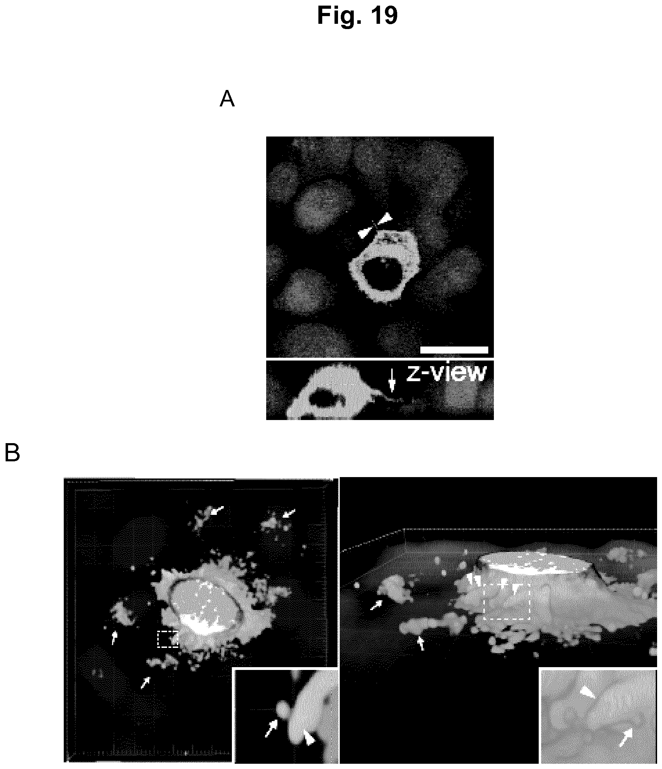

- Figs. 19 A-B: are a single confocal plane image (A) and a 3D reconstruction (B) of an transferrin mCherry loaded donor cell which interacts with an acceptor cell through tunnelling nanotubes (arrowheads) whereby vesicles are transferred into acceptor cells (arrows);

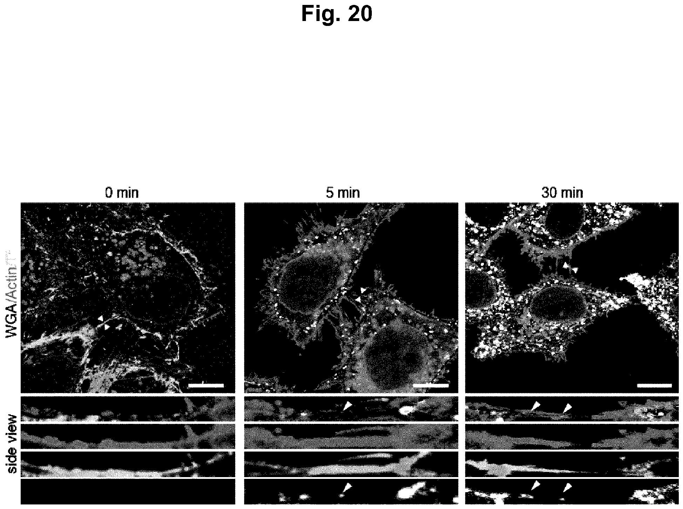

- Fig. 20: are micrographs of TNTs in HeLa-Kyoto cells using wheat germ agglutinin and actin staining which show transferrin-ligand positive endosomes 5 and 30 minutes after ligand internalization;
- Fig. 21 A: is a table with a numerical representation of the contact-dependent cell-to-cell transfer quantitation upon treatment with different short interference RNAs (siRNAs);
- Fig. 21 B: is bar diagram showing the integrated DiD transfer intensity/volume ratio upon treatment with different siRNAs.

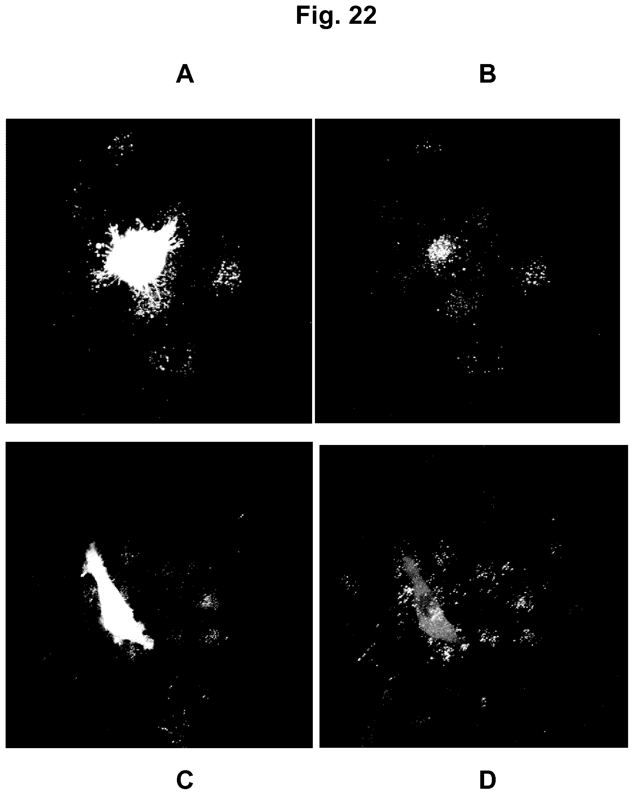

- Fig. 22A-D: are microscopic images of donor cells comprising both fluorescent DiD and myosin-X (Myo10) or a mutant form of myosin-X (Myo10-heavy meromyosin) - (A) DiD channel; (B) EGFP_Myo10 (EGFP - enhanced green fluorescent protein); (C) DiD channel; (D) EGFP_Myo10-HMM (EGFP channel)

### DETAILED DESCRIPTION OF THE INVENTION

For the purposes of the present disclosure, the terms shall have their usual meanings unless defined as herein below:

*Contact-dependent cell-to-cell transfer* comprises substance transfers mediated by a cell structure formed between cells *in vitro* and *in vivo.* TNTs are for example *de novo* formed cell bridges that facilitate a transfer of intracellular ions, proteins, vesicles and organelles between cells. The antonym is medium-mediated transfer which requires an extracellular fluid or medium. Contact-dependent cell-to-cell transfers should be vectorial and directed.

*Singularized cells* are cells where all cell-cell interactions have been disrupted, usually by enzymatic digestion of surface proteins and/or other biochemical interruption of cell-cell adhesion, and thereafter reinforcing detachment mechanically by pipetting, squeezing or vibration. After this procedure cells will attach to a substrate as single cells. The antonym to singularized cells is clusters of cells or a cell tissue. "Singularized cells" or "individualized cells" may be obtained by disrupting clusters of cells by mechanical stress and/or enzymes used for preparing a primary cell culture. Clusters of cells of a confluent cell culture can be made singular by forcing the cell clusters through the opening of a small pipette.

*Region of Interest (ROI)* is a three-dimensional space volume comprising an identified donor cell and a multiplicity of surrounding acceptor cells or only acceptor cells. The region of interest (ROI) is preferably twice the size to cover the entire contact-dependent cell-to-cell transfer that may have occurred. The ROI space preferably has a side length of about 200 micrometers or more in xy and a vertical axis (z-axis) to cover the full height of the cells under examination, usually 10 to 30 µm. The region of interest may consist of multiple integrated planes in the z-axis (typically about 20-30 of 1 micrometer or less) while a 3D confocal scanning microscope is preferred for its higher accuracy. The region of interest typically corresponds to the widest field of view of a standard confocal microscope at 40x magnification.

*Traceable* are materials or substances having a detectable marker or labeling. The marker or labeling may be a fluorescent dye or a fluorescent protein such as the (enhanced) green fluorescent protein or mCherry. The traceable material may be expressed transiently or constitutively in the donor cells. Alternatively, a protein expressed in the donor cell only may be detected by immunohistochemistry or radioactive labelling. Further, the detectable marker or cell labelling may be a lipophilic tracer such as long-chain dialkylcarbocyanines, in particular Dil or DiA, and dialkyl aminostyryl dyes (DiA and its analogs), labelled wheat germ agglutinin (WGA) or another organelle tracer.

*Imaging or staining for cell microscopy* comprise methods of surface staining of donor and acceptor cells to make them accessible to cell microscopy and whole cell segmentation of both single cells and clustered cells. This can be done for example by a staining with wheat germ agglutinin coupled to a fluorescent dye (e.g. Alexa-Fluor 488) or by any other suitable dye (long-chain dialkylcarbocyanines, in particular Dil or DiA, and dialkyl aminostyryl dyes (DiA and its analogs),) or a labeled binding molecule suitable for microscopical image acquisition. Cell detection can be improved by adding a marker defining a structure within each cell, e.g. Hoechst or DAPI staining. The cells may be stained as described in the examples while numerous other methods are available to the person skilled in the art.

*Cell segmentation* is the process of separating imaged cells from the background and from other cells. Whole cell segmentation is the process of further providing information about the location of the total volume of each cell and can thereby provide information about cell characteristics affiliated with individual cells such as volume, shape, signal distribution, neighborhood relations and cell movements over time. Automated cell segmentation and analysis of imaged cells provide more objective data and thereby enhance reproducibility. Cell segmentation can also be applied to unstained cells which minimizes the disturbance of live cells due to the lack of the chemical influence of a dye and due to a reduction of phototoxicity (see Sacan et al, Bioinformatics 2008, 24(14):1647-1649; Tscherepanow et al, Advances in Mass Data Analysis of Images and Signals in Medicine, Biotechnology, Chemistry and Food Industry (MDA 2008): Springer Lecture Notes in Computer Science Volume 5108; 2008:158-172; Wu et al IEEE Trans Biomed Eng 1995,42:1-12; Yu D et al, J Neurosci methods 2007, 166:125-137. citeseer.ist.psu.edu/wu95live.html).

*Proliferation inhibitors* are agents essentially inhibiting any further cell proliferation such as deoxythymidine ("thymidine"). The proliferation of mammalian cells in cell culture can also be inhibited by using a serum-depleted culture medium or a combination of cell proliferation inhibitors such as apicidin™, sodium 4-phenyl-butyrate, trichostatin A and valproic acid which are all cell permeable inhibitors of histone deacetylase. Proliferation inhibitor does not mean that the proliferation of cells is completely stopped but that the division of cells is reduced to a remainder of 10 to 20 percent divided cells which underwent division during the first hour before adding the inhibitor or by a leakiness of the inhibition process.

*Quantitative analysis* means herein that the amounts of cell-to-cell substance transfer are determined using the method described. It is obvious to further analyze the cell-to-cell transfers as to speed, direction, and distribution of material transfer.

*Voxel* is a volumetric pixel or volumetric picture element which represents a gray value on a regular grid in three dimensional space and can be acquired in several color channels representing different markers. Voxels do not typically have their position (their coordinates) explicitly encoded. Instead, the position of a voxel is inferred based upon its position relative to other voxels and within the predefined region of interest (ROI). While voxels are frequently used in the visualization and analysis of medical and scientific data, in the present disclosure the acquired twodimensional (e.g. 512 x 512 pixels of 0.445 µm in xy) images are analyzed in consecutive microscopic planes (e.g. 1.01 µm) as to the integrated fluorescence or staining intensity over background in preset cell volumes. The analysis of the images is preferably done automatically as published by Hodneland et al., CellSegm - a MATLAB toolbox for high-throughput 3D cell segmentation. Source Code for Biology and Medicine 2013,8:16.

*Confluence* in the present context corresponds to the density of donor and acceptor cells cultured on a microscopically compatible substrate. When cells are grown in a cell layer ideally not leaving any substrate uncovered, the cells are confluent. In the example of HeLa-Kyoto cells, a final density of up to 35 000 cells per square centimeter corresponds to a confluent cell layer. In the present method, a homogenous cell suspension is produced containing donor and acceptor cells in a ratio from preferably 1:100 to 1:2000 (or higher when applied to 3D cell cultures), and then the cells are plated on a substrate (or into a matrix when applied to 3D cell cultures) with a density to obtain donor cells in a lawn (or sphere when applied to 3D cell cultures) of acceptor cells. The "cell culture" may also be in a host animal via an implant or transplant or even an artificial tissue. A suitable ratio therefore depends on the desired cell density, the size of the cells, the viability of the cells and experimental needs as the proliferation of cells is inhibited. Preferably and for reliable quantitative analysis, single donor cells are plated such that they end up mostly in contact with at least four or more acceptor cells and wherein the number of cells in a region of interest (ROI) is 18 or more, most preferably 20 or more. Consequently, the donor and acceptors are not only combined in a certain ratio but they are further seeded and cultured on a microscopically compatible substrate with a density corresponding to confluence for the sake of efficiency, reproducibility, minimized variation and therefore meaningful biological results.

The present application for a patent relates to a microscope based method for quantitative analysis of contact-dependent cell-to-cell substance transfer between cells, preferably cells of vertebrate or mammalian origins. The method comprises the preparation of a homo- or heterocellular mixture or combination of donor and acceptor cells wherein the donor cells comprise a unique traceable substance. Typically, the donor cells are combined with acceptor cells in a ratio so that donor cells are statistically surrounded by only acceptor cells within a region of interest. The culture step secures the *de novo* formation of cellular structures between single cells on a substrate. As mentioned, said donor and acceptor cells may be of same or different. type. The cells may be chosen from primary tissue cells, established cell lines, cancer cells, stroma cells, virus-infected cells, cells and cell lines of the immune system, artificial cells. The disclosed method can be used with any adherent tissue, primary cells or cell lines. Cells and cell lines of mammalian and vertebrate origins are preferred for clinical, veterinarian and pharmaceutical studies.

The disclosed method is best used together with a confocal microscope for producing fluorescence image stacks compatible with whole cell segmentation and analysis of data on individual cells such as volume, shape, signal distribution, neighborhood relations and cell movement over time. Images of consecutive microscopic planes are analyzed as to the intensity of traceable substance over background. Thereby the bulk of traceable substance is directly determined for each volume element. It is preferred to further analyze identified substance transfers for their vectorial characteristics. However, any fluorescence microscope may be used. It is just that confocal or spinning disc and/or 3D increase the accuracy of the described method.

A representative embodiment of the method comprises the following steps. Obtaining of a population of single cells which have got loaded by phagocytosis, pinocytosis or endocytosis or microinjection with a fluorescent stain, for example, a fluorescent long-chain alkyl carbocyanine. The cells are then washed to remove any excess of stain. In another embodiment, the donor cells express transiently or constitutively a fluorescent protein, for example, a fusion of the enhanced green fluorescent protein and myosin-X (EGFP-Myo10) or GFP-transferrin.

A second population of single cells is obtained which are assigned to be acceptor cells. The acceptor cells may contain no stain nor fluorescent labeling. If the acceptor cells comprise a labelling or a marker protein, then the fluorescent excitation or emission spectra should be different to the fluorescent marker or dye used for labelling the donor cells.

The traceable substance or material may be selected from one or more of the group consisting of dyes, fluorescence dyes, DiD, Dil, DiO, Lysotracker™, Mitotracker™, radioactive marker substances, luminescent dyes, fluorescent dyes, electroluminescent dyes, fluorescing proteins, luminescing proteins and peptides, nanoparticles, quantum dots (QD). The traceable substance may be selected from one or more biological materials of the group consisting of bacteria, parasites, viruses, subviral agents, viroids, virusoids, satellite viruses, prions; lentiviruses, adenoviridae, herpesviridae, poxviridae, papovaviridae, parvoviridae, picornaviridae, annelloviridae, togaviridae, retroviridae, human immunodeficiency virus (HIV), rhabdoviridae, orthomyxoviridae, hepadnaviridae; labelled cell organelles, labelled cell vesicles, protein aggregates (Huntingtin, Tau, parts of tau), mRNA, microRNA, DNA, nanoparticles.

Accordingly, the contact-dependent cell-to-cell substance transfer may be associated with a transmission and spreading of infectious or pathogenic materials. The infectious or pathogenic labelled material may be from group consisting of double stranded DNA, singe-stranded DNA, double-stranded RNA, (+)single-stranded RNA, (-) single-stranded RNA, bacteria. It is contemplated to use the disclosed method for studying the cell-to-cell transmission of HIV and prion diseases. Another aspect of the invention is associated with a screening of pharmaceuticals and active agents for activity against viral, bacterial and parasitic diseases transmitted by contact-dependent cell-to-cell transfer and molecular communication.

A further aspect relates to a screening of compounds for use in the treatment of tumors, metabolism disorders, nervous system disorders, circulatory disorders, and in the field of cell/tissue culture and animal models. The method can also be used in the testing of drugs and compositions in gene therapy, cell targeting and pharmacology.

As the traceable substance may be a relevant protein, nucleic acid, an organelle or a pharmaceutically active compound, the microscope-based method can be used to analyze cell-to-cell spreading of infectious agents, diseases and drugs. In other words, the method can not only be used for a screening of drugs but also for a monitoring the cell-to-cell transfer of a drug already targeted to a specific group of cells. Such a quantitative analysis of cell-to-cell transfer is considered particular important in the field of cancer therapy as cancer cells interact with surrounding stromal cells, immune cells, blood and lymph vessels, fibroblasts and those are targets for fighting cancer progression and invasion.

The method becomes more precise when the contact-dependent cell-to-cell transfer are not just quantified but also analyzed for vectorial distribution. A vectorial analysis of substance transfers excludes specifically any non-vectorial, medium-based substance transfers. The method can also be used with pairs of one donor cell and one adjacent acceptor cell.

In the examples hereinafter there is a step of embedding donor and acceptor cells in a three-dimensional matrix such as low-melting point agarose. Such a matrix is typically used to inhibit medium-mediated cell-to-cell transfers. As the disclosed method can per se distinguish medium-mediated from contact-dependent cell-to-cell transfers a culturing of cells in low melting point agarose is usually not needed or for control purposes only.

The present disclosure suggests studying contact-dependent cell-to-cell transmission of HIV and HIV protein, prion proteins and prion disease, Huntingtin aggregates, Huntington disease. Likewise, cell-to-cell transmission of poliovirus and hepatitis C viruses as cell permeabilization by poliovirus triggers bystander permeabilization in neighbouring cells (Madan et al, Cell Microbiol. 2010 Aug;12(8):1144-57). Hepatitis C virus on the other hand can evade the immune system and has a history of chronic development.

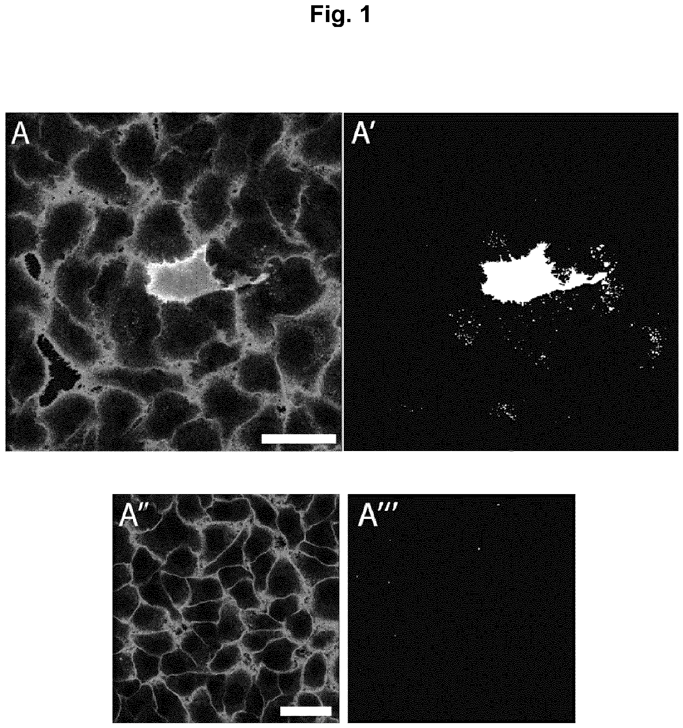

A quantitative analysis of cell-to-cell transfers not only requires high resolution imaging but means and steps which allow differentiation between medium-mediated and contact-depending cell-to-cell transfers and transmissions. This differentiation is achieved by a microscope-based analysis wherein the object contains spaced apart donor cells cultured in a lawn of acceptor cells so that contact-dependent cell-to-cell transfers can be tracked reliably and with high accuracy when simultaneously the cells on the substrate and their position are capture by cell segmentation. A whole cell segmentation even allows a vectorial analysis of the cell-to-cell transfers and the elimination of medium-mediated background transfer to below 5 percent for cell dyes such as DiD and to even lesser amounts for expressed fluorescent transfer proteins as shown by Figures 1 to 3. The results obtained in the prior art by microscopy and flow cytometry were not comparable (Bukoreshtliev N.V. et al., FEBS Lett. 2009, 6;583(9):1481-8). One reason for this was that donor and acceptor cells were mixed and cultured in a ratio that in acquired microscopic images it could not be detected which cell the cell of origin of the transferred material was.

A person skilled in the art will appreciate that donor and acceptor cells must be plated on a suitable substrate in a sufficiently high controlled density, ideally with a density close to confluence in order to optimize cell-cell contact opportunities. When the cells have attached to the substrate, a proliferation inhibitor is added to the culture medium, or the medium is replaced to one inhibiting proliferation, e.g. by as reducing serum or growth factor content. A widely used proliferation inhibitor is thymidine, more precisely deoxythymidine. Addition of a cell proliferation inhibitor provides against cell division of the donor cells, and also provides against cell overgrowth. After addition of the proliferation inhibitor the donor and acceptor cells are allowed to interact for a period of time to allow for cell-to-cell transfer and transmission.

The instant method is superior to a quantitative analysis based on flow cytometry since the latter cannot distinguish between contact-dependent substance transports and medium-related processes, including an uptake from apoptotic cells. As the instant method can in each case allocate the substance transfer to a single donor cell, the investigator has further parameters available such as the vectorial direction of the substance transfer, the average cell distances, the bulk of transferred material, the status of the cells and of course the physiological role of the transferred proteins.

The present disclosure further relates to an automated method for an evaluation of large amounts of microscope data facilitating high throughput screenings. This can be achieved by automating the image uptake wherein a computer operates the microscope with the help of a pattern recognition program. The 3D datasets are then segmented by CellSegm and analyzed by another software producing the data read-out in numbers, suitable to statistical analysis. The transfer amount, density, direction can be read out for every individual cell in the culture and correlated to expression levels, shape, size of all acceptor cells and the corresponding donor cell and distances and other relations between them. It is further contemplated using a three-dimensional cell culture model to imitate a physiological tissue.

For studying cell-to-cell organelle transfer, a differential staining of donor and acceptor cells may be used. Thus, the cell membranes of all cells may be stained with AlexaFluor™-conjugated wheat germ agglutinin (WGA-AF488) and the organelles of the donor cells either with a lipophilic tracer, e.g. long-chain (C18, C16 or C12) dialkylcarbocyanine such as DiD, DiO, Dil (Invitrogen) or a dialkylaminostyryl dye (DiA) or vice versa. This approach allows the visualization of DiD-positive organelle transfer from donor cells into WGA-AF488-positive acceptor cells, or vice versa. Alternatively, the organelles may also be with a fluorescent protein such a EGFP-Myo10. This is proposed because myosin-driven intercellular transportation of wheat germ agglutinin is known to be mediated by membrane nanotubes between human lung cancer cells (Wang et al, ACS Nano. 2012 6(11):10033-41. doi: 10.1021/nn303729r) and because EGFP-Myo10 is transferred massively between HeLa Kyoto cells (Kögel T, Frei DM et al, unpublished data).

The results obtained by flow cytometric analysis suggest that the percentage of cells receiving material increases with cell density for a constant ratio of donor and acceptor cells (Fig. 7 A). The results obtained with the present method however suggest that a substantial portion of the previously measured intercellular (DiD) transfer is not by contact-dependent mechanisms or TNTs. Flow cytometric analysis for measuring contact-dependent material (DiD) transfer in high-density 1:1 co-cultures is prone to measure medium-mediated types of transfer.

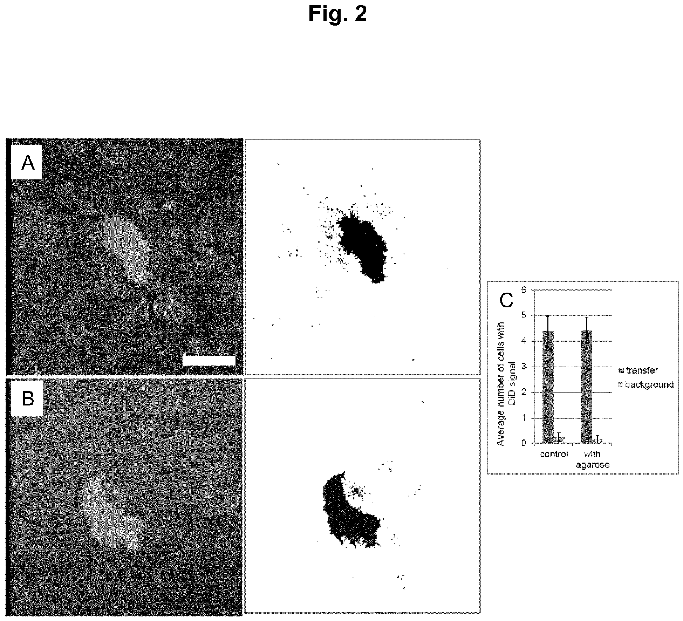

This highlights the importance of the feature that the ratio of donor to acceptor cells needs to be low. A ratio of donor to acceptor of 1:400 allows the analysis whether the observed substance transfers possesses a vectorial direction. This can be done only when a single donor cell is plated in a larger group of surrounding acceptor cells; see Figs. 1A-A''' and Fig 2C. In these figures all cells were stained using WGAAF488 and the donor cells have DiD stained organelles. The respective cell lawn has further a very low background wherever no contact-dependent transfer has occurred. A distance of 200µm or more from each DiD donor cell was used for our screens with Hela Kyoto cells when acquiring images for background analysis. If other cell types are used, the maximum transfer distance/time needs to be assessed. As intercellular substance transfers occur more frequently in low amounts, a low background is favorable for high resolution imaging and vectorial analysis and for the precision of the resulting data and detection limit for effects of the investigated agents. With the shown set-up, we could observe that more than 75% of the plated donor cells made physical contacts to surrounding cells and intercellular material (DiD) transfers within 12 hours after plating. However, after 20 h a more robust readout was possible due to larger amounts of transfer that occurred within the longer timespan.

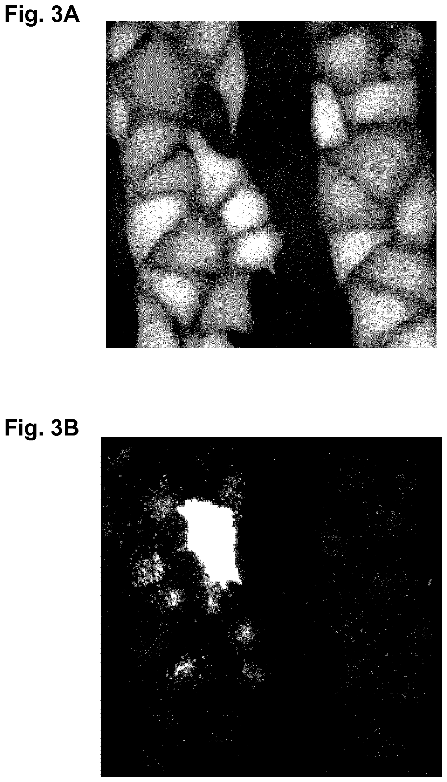

A low background further allows an analysis of the pattern of substance transfers. The contact-dependent substance transfer occurs not at random, say in circular diffusion-like pattern around the donor cell but in our observations regularly in preferred direction (Fig. 1 A' and Fig. 2 B-B'). Any directionality of the substance transfer confirms that the transfer is based on a transient cell structure. The experiments with cells embedded in low melting point agarose support this notion. Likewise, when cells are grown such that they are surrounded by non-adhesive areas or stripes, then no significant intercellular substance transfers can be observed; see Figs. 3A, B. A substrate can for example be made non-adhesive to cells when coated with a protein such as bovine serum albumin and plating exclusively on the adjacent adhesive stripes is facilitated by seeding cells in a microfluidic pattern.

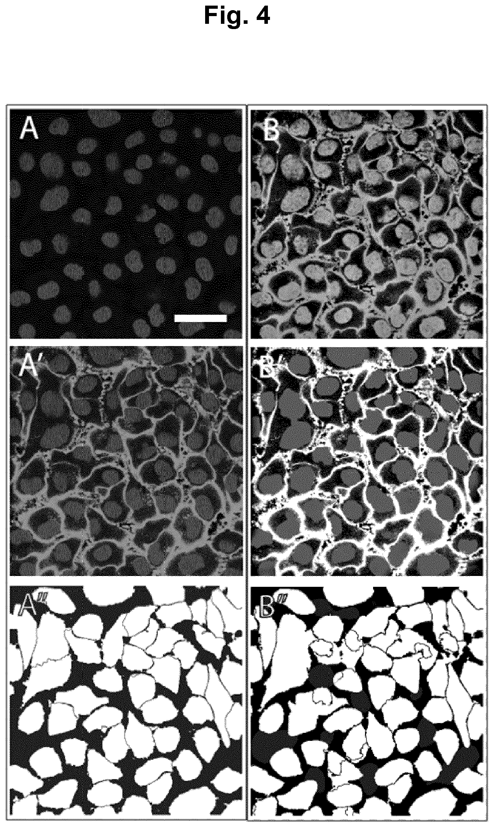

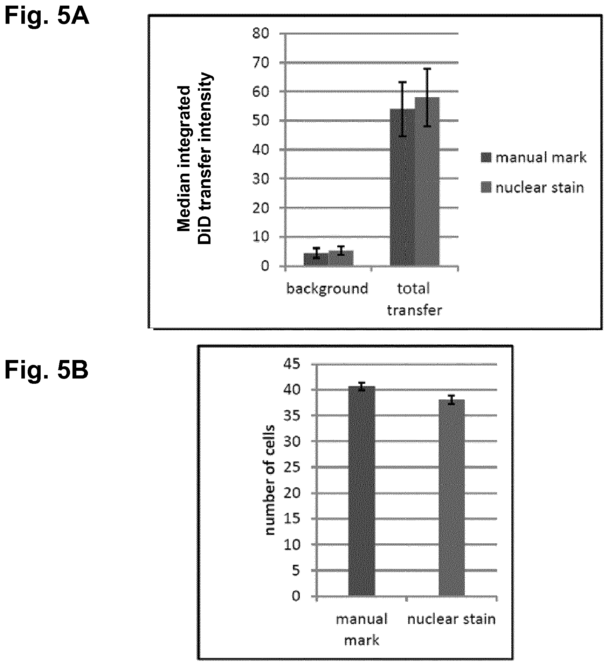

The present disclosure further relates to a 3-dimensional (3D) confocal microscopic analysis of cell contact-dependent transfer in order to capture the absolute amounts of transferred material. In detail, the method comprises the acquisition of 3D image stacks approximately at 1µm z-distance followed by a segmentation of cells in the channel corresponding to the stained cell membrane. The acquired images can for example be processed by anisotropic filtering, marker regeneration, watershed segmentation and final classification of the watershed regions into background or cells. The marker-controlled watershed segmentation as disposed herein on the presence of one single marker per cell, which can be assigned i) manually or ii) automatically, for example based on Hoechst nuclear staining; see e.g. Fig. 4 A-B"). To analyze material (DiD) transfer, the fluorescence signal (DiD) above background level can be quantified as a read out for signal (DiD) acquisition within the computed borders. The measured fluorescence (DiD) integrated intensity per cell then yields very similar values by following any of both approaches (see example in Fig. 5C, D). Thus the disclosed method can advantageously provide an automated quantification of the contact-dependent cell-to-cell transfer. This automated approach enables the investigator to analyze a large number of samples under different conditions, satisfying the requirements of accurate signal quantification and traceability of the cell-to-cell substance transfers.

This has been exemplified for the transferrin receptor, which is a well-known marker of recycling endosomes. We fused the transferrin receptor to a fluorescent protein (mCherry) and transfected HeLa-Kyoto cells with it a plasmid encoding for this fusion protein. Donor and acceptor cells were co-cultured in low donor/acceptor ratio co-cultures and at confluence, as described above.

In detail, the donor population was either transfected with the transferrin receptor-mCherry or mCherry alone as control. The acceptor cell population was stained with CellTracker™Blue (CTB), which emits fluorescence and is not transferred to adjacent cells in the population. These two differentially labeled cell populations were co-cultured at a donor/acceptor 1:200 ratio in a confluent monolayer. To evaluate the protein transfer, we compared the amount of mCherry signal to the CTB positive voxels within the region containing acceptor cells surrounding single donor cells, which were expressing Tf-RmCherry or mCherry alone, by microscopy. We observed that the transfer of the transferrin receptor was threefold increased compared to mCherry alone. (Fig. 13B, C). Furthermore, acceptor cells located distantly to donor cells displayed low transfer rate of Transferrin receptor. We therefore show that the transfer of proteins is specifically controlled and does not take place by a passive mechanism upon co-culturing according to the method of the invention.

We continued to investigate the nature of such protein transfer mechanism by treating CTB-stained cells with conditioned medium obtained from cells transfected with Transferrin receptor or mCherry alone, which were compared to cells cultured in normal medium as control. Considering that the fluorescence levels of the treated CTB-stained cells did not exceed background levels (Fig. 17A, B), we excluded the possibility of microvesicle/exosomal-mediated intercellular transfer in our experimental conditions.

By means of seeding cell co-cultures with PDMS micro-patterned surfaces and live cell imaging, we showed that cells physically separated from each other by 15µm or further do not incorporate Transferrin receptor (Fig. 18). We found that, similarly as in the already described DiD experiments, the transfer of proteins between cells in direct contact seem to involve the formation of protrusions arising from the donor cell. Notably, we could observe these protrusions extending towards the acceptor cell and inserting within the CTB-signal structures (Fig. 19 A). The formation of intercellular bridges and the presence of Transferrin receptor along them, strongly suggests the participation of tunneling nanotubes in this contact-dependent transport mechanism. However, it was not clear whether the transfer occurs by uptake of plasma membrane from the tube or by formation of organelles, encapsulating the transferred proteins. To answer this question, the ligand of the Transferrin receptor was coupled to a fluorophore and let be internalized by the cells. The presence of transferring spots in the tunneling nanotubes in a time-dependent manner, suggested the use of these bridges by early and recycling endosomal-derived vesicles (Fig. 20), favoring the notion of organelle-mediated protein transfer across TNTs.

This intercellular communication fashion is therefore similar to the proposed cell-to-cell transmission of pathogens in human cells (Abela et al., PLoS Pathog. 2012, 8(4):e1002634; Monel at al., J Virol. 2012, 86(7):3924-33). Correspondingly, we assumed that the method of the invention enables the monitoring and quantification of cell contact-dependent transfer of pathogens, for example, viral particles, in unprecedented resolution and accuracy.

The Human Immunodeficiency Virus (HIV) type-1 is known to propagate in two different modes: a) infection by cell-free virions and b) direct cell-to-cell transmission of the virus. Cell-to-cell transmission has been described as a more rapid and efficient mechanism than in cell-free mode, such that it enables avoiding several biophysical, kinetic and immunologic barriers. Cell-to-cell infection requires the physical interaction between the infected cell and the HIV receptor-bearing surface of target cells, resulting in the formation of the so called "virological synapses". Interestingly, HIV cell-to-cell transmission requires the assembly of fully infective particles for this transmission to take place. Virus transmission *in vitro* has been proven to be more efficient through these points of contiguity than infection by-cell-free viruses, supporting the notion that cell-to-cell transmission may be a relevant if not a dominant mode of virus dissemination in infected individuals (Abela et al., PLoS Pathog. 2012, 8(4):e1002634; Monel at al., J Virol. 2012, 86(7):3024-33). So far, the relative contribution of each of the transmission modes in HIV infection and dissemination remains unclear. Other studies further suggest that HIV-infection might enhance the formation of tunneling nanotubes in macrophages and also that viral particles may be transported across these intercellular communication structures as a mode of direct cell-to-cell transmission of the HIV virus (Eugenin et al., Commun Integr Biol. 2009, 2(3):243-4).

The design of effective therapies is conditioned by this lack of information. If cell-to-cell transmission indeed represents a primary mode of infection and spread, the use of neutralizing antibodies or viral inhibitors may not lead to the desired effects. Since the viral particles prevent their exposure to inactivating substances by remaining at all times within protected areas, they will inevitably escape for the activity of the therapeutic approaches directed to cell-free viral transmission *in vivo.*

McDonald et al. (J Cell Biol. 2002,159(3):441-52) describe that "the majority of HIV particles that are not associated with microtubules are in the peripheral regions of the cytoplasm" and therefore in close proximity to the cell membrane. Without wishing to be bound to a theory, this phenomenon seems consistent with the fact that tunneling nanotubes-mediated transport in many investigated cases does not involve microtubule-activity. This means that upon cell-to-cell transfer, the HIV virions must adopt an alternative mode of motility.

We propose here a method, by which cell-contact dependent transfer of material, for example by means of naturally occurring tunnelling nanotubes (TNTs), can be monitored and accurately determined. Indeed, transport along TNTs has been proposed for viral propagation between mammalian cells. In particular, this could explain why the human immunodeficiency virus (HIV) can escape the neutralization activity of specific antibodies against its component viral proteins (Abela et al., PLoS Pathog. 2012, 8(4):e1002634). The method of the invention evaluates the ability of TNTs for directing transport between human cells, and therefore allows a determination of the infectivity of such virus and other pathogens propagated by cell contact-dependent transmission.

### EXAMPLES

### Example 1- Determination of contact-dependent cell-to-cell organelle transfer

### a) Cell culture, staining and co-culture

HeLa-Kyoto cells were cultured in DMEM:F12 supplemented with 10% fetal calf serum and 1% penicillin/streptomycin (Invitrogen Detection Technologies, Carlsbad, CA, USA) on 24-well microscopy plates (Greiner Bio-One GmbH). Cells were maintained under humidified air supplemented with 5% CO₂ at 37°C and plated with a density of approximately 35 000 cells/cm², After 24 hours, 200ng (5µl)/ml lipid analog Vybrant® DiD Cell-Labelling solution (Invitrogen Detection Technologies, Carlsbad, CA, USA) was added to the plated cells in culture medium, incubated at 37°C for 20 min wherein tilted 5 times. After the incubation period, the culture medium was exchanged 4 times within 2 hours. Donor DiD-stained cells were washed twice with phosphate buffered saline PBS (Sigma-Aldrich, St. Louis, MO 63103, USA) and harvested by trypsinization (0,25% trypsin/EDTA) (Sigma-Aldrich, St. Louis, MO 63103, USA) for 10 min. Trypsin activity was neutralized by addition to the cells of 5 ml culture medium. Subsequently, cells in solution underwent centrifugation at 129 g and the supernatant was removed by pipetting it out, leaving approximately 100µl liquid/pellet. 2ml fresh medium was added to the cell pellet and by pressure pipetted for 15 times with a pipette Pasteur, cells singularized. Donor DiD-stained cells were either a) co-cultured 1:1 with non-stained acceptor cells or b) diluted 1:400 and then co-cultured with unstained acceptor cells for 2 hours. After this incubation time, the culture medium was exchanged to a medium containing 2mM deoxythymidine (dthymidine) (Sigma-Aldrich, St. Louis, MO 63103, USA) and 100mM cytochalasin D (Sigma-Aldrich, St. Louis, MO 63103, USA) or no serum if indicated. The mixture of DiD-stained donor cells and non-stained acceptor cells was cultured for additional 18 hours. The total co-culturing time represented therefore 20 hours, followed by either direct imaging of living cells or fixation, staining and imaging of fixed cells.

### b) Cell fixation and plasma membrane staining

In order to fix the cells, co-cultures of donor and acceptor cells were washed with PBS for 1 min followed by incubation with 4% paraformaldehyde (PFA)/4% sucrose/PBS (Sigma-Aldrich, St. Louis, MO 63103, USA) for 35 min. Afterwards, a NH₄Cl (50mM)/PBS solution (Sigma-Aldrich, St. Louis, MO 63103, USA) was added to the co-culture and further incubated for 2 min. Then, washed with PBS for 1 min.

For staining of the plasma membranes and cell nuclei, a solution containing 500ng/ml AlexaFluor™-conjugated wheat germ agglutinin (Invitrogen Detection Technologies, Carlsbad, CA, USA) (WGA-AF; membrane) and 4µl/ml Hoechst 33342 (Sigma-Aldrich, St. Louis, MO 63103, USA) (nuclei) in PBS was added to the coculture and incubated for 10 min. To stop the staining process, the fixed co-culture was washed for 1 min with PBS. Optionally, the cells underwent a second fixation step in order to avoid strong staining of the intracellular membrane systems. The resulting fixed and stained co-cultured cells proved to be valid for microscope image acquisition of segmentation quality for at least 9 days when stored in PBS at 4°C. For longer storage periods, 0.02% sodium azide (Sigma-Aldrich, St. Louis, MO 63103, USA) was added.

### c) Cell imaging

Cells were imaged with a Leica confocal SP5 in resonant scanner mode (zoom 1,7; pinhole air1; 40X 1,25NA oil immersion objective; 512 x 512 pixel; z-distance 1,01µm, line-average 16; offset -1; gain 900-1000V. For the DiD-channel, laser power was adjusted for each experiment to a strength resulting in approximately half a cell diameter of out-of-detection range data for average donor cells, in order to quantify the transferred DiD in the surrounding cells, present in much lower quantities. For all other channels, the laser power was adjusted to few out-of-detection range pixels at the upper end of the scale.

### d) Automated image analysis: cell segmentation

Based on recent findings in which DiD stained organelles have been shown to be transferred between cells, we set out to establish a system to document its contact-dependence. By seeding DiD-stained donor and unstained acceptor HeLa-Kyoto cells at a ratio 1:400 and confluent density, DiD transfer could be allocated to specific single donor cells. The following design allowed the establishment of a quantifiable read out for intercellular transfer.

All programs for segmentation and automated analysis were written in MATLAB. The 3D image stacks were acquired approximately at 1µm z-distance and the cells were segmented in the WGA-AF channel including the processing steps of anisotropic filtering, marker regeneration, watershed segmentation and finally classification of the watershed regions into background or cells (see Hodneland et al. and Fig. 4). The segmentation method is using marker controlled watershed segmentation, relying on the existence of a set of markers or initialization regions, with exactly one marker for each cell. Markers were defined by two methods which did not yield significantly different results if applied to the same dataset: (i) for semi-automated segmentation, markers were assigned manually assigned in the WGA-AF images and used as seeds for the segmentation. Separate markers were given for cells and background, which in combination were used for segmentation and high-quality cell classification. (ii) For fully automated segmentation a nuclear staining from a separate channel was used to generate markers inside the cells, which were used as seeds for segmentation in the WGA-AF channel. To achieve this, the nucleus channel was iteratively thresholded until the largest thresholded object exceeded the minimum predefined nucleus volume, corresponding to an ellipsoid with a volume of 839µm³. Small regions below the volume of an expected volume were removed, and all objects were closed to connect objects belonging to the same nucleus which were closely situated but erroneously disconnected. Gaps were filled and, to separate erroneously connected nuclei, objects cut into several objects if they had several peaks at the higher sections. Background markers were also defined by iterative thresholding until the largest object exceeded the maximum expected cell size, with a volume corresponding to a sphere with a diameter of 130 voxels and no overlap to the already found cell markers. Otherwise, the second largest background marker with no overlap to nucleus markers was used. After watershed segmentation, the cells were classified using information from the detected nucleus markers, such that an object with an interior nucleus marker was assigned as a cell and not as background.

To analyze DiD transfer, DiD signal above background level (100 of 256 greyscale intensities) was quantified as a read out for DiD transfer within the computed borders. The objects above the expected cell volume were classified as cells (volume corresponding to a sphere with a diameter of 20 voxels) and the outer boundary of the DiD-cell was smoothened by top-hat filtering. In Fourier space, a top hat filter selects a band of signal of desired frequency by the specification of lower and upper bounding frequencies. The program automatically detected when the image was a control image or not, depending on whether a DiD donor cell could be detected or not. The signal contained in the computerized cell volumes was defined as cell-to-cell transfer. Unspecific transfer was all DiD signal above threshold 100 outside of the cells.

In essence, the greyscale intensity of each voxel within the acceptor cells found in a region of interest was measured (256 greyscale intensities from 0 to 255). Greyscale intensities below 100 were set zero and ignored. All greyscale intensities were added up for each cell in case of a vectorial analysis or region of cells. With respect to the number of acceptor cells surrounding a donor cell, we further defined that a region of interest only qualifies as a region of interest (ROI) when it contains at least twenty cells whereof at least four acceptor cells must be in contact with an identified single donor cell in order to achieve data with minimized variation and thereby increased reproducibility. This further definition of the region of interest is arbitrary in that other reasonable pairs of cell density and contact cell numbers are possible, e.g. 30/6 to 10/1 but it should be obvious that a cell-to-cell transfer cannot take place in a region of interest which contains no cells or no cells in contact with the donor cell. There is also a physical upper limit as to the number of acceptor cells which can contact a single donor cell.

Contact-dependent cell-to-cell transfer as measured according to the described method results in entity sizes with a log-normal distribution as typical for natural processes independent from initial value or concentration. In the present case, the traceable substance must be present in the donor cells above a certain threshold. Consequently, the quantitative measurement is either expressed relative to a control condition or to the expression in the donor cell or both. The bounds of this random sampling process is therefore described by the standard error of the means. Whether or not the measured values are statistically significant is determined after logarithmic transformation to obtain a continuous probability distribution. Statistically significantly different sets of data are detected by the t-test (Student's t-distribution, unpaired, unequal variance" (p <0,05) in case of two similar data sets (background vis-a-vis cell-to-cell transfer). Otherwise, the sets of data can be analyzed using ANOVA and the post-hoc Bonferroni-Holm test (p <0,05) or the false discovery rate (FDR) in case of multiple hypothesis testing.

### e) Assignment of DiD transfer to individual donor cells

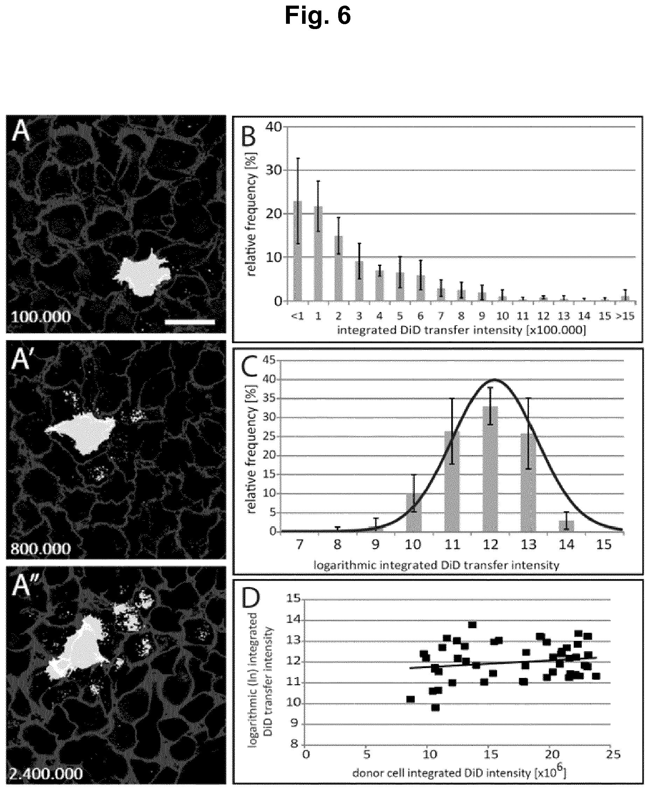

By seeding DiD stained and unstained HeLa-Kyoto cells at a stained/unstained 1:400 ratio and confluent density, the DiD transfer could be allocated to specific single DiD-positive donor cells, enabling the establishment of a robust and quantifiable read-out for intercellular transfer (Fig. 1 A-A''' and Fig. 2A, B). Staining the membranes with AlexaFluor™488-conjugated wheat germ agglutinin (WGA-AF-488) allowed distinguishing the number of acceptor cells and quantifying the amount of DiD that each individual cell received (Fig. 1 A-A' and Fig. 2 C). The background in imaged areas of the same culture well at a 200µm distance or further from any DiD-stained cell was very low (Fig. 1 A", A''' and Fig. 2 C). Notably, low amount of transfer was more frequent than high transfer, thereby enabling a better discerning of the transfer (Fig. 6A, B). The relative frequency of transfer intensity was log-normal (Fig. 6 C). Within the applied parameters, the integrated DiD signal intensity of the donor cell and of the total transfer around the donor cell did not correlate (Fig. 6 D). The background dataset displayed a lower overall and average intensity compared to the total transfer dataset (Fig. 5 A). But each transfer material region had a higher intensity in the background regions compared to the transfer regions (Fig. 11B). Almost 80% of the acquired images containing a donor cell (which itself was excluded from the measurement) displayed higher signal intensity than 95% of control acquired images. Less than 25% of the acquired images with a donor cell had integrated DiD signal intensities as low as in control images. Therefore, more than 75% of donor cells transferred DiD above background levels within 20 hours.

### f) Directed non diffusion-mediated mechanism of transfer

Importantly, the transfer pattern was usually not oriented in an even circular scheme around the donor cell (Fig 1A', Fig. 2A, B and Fig. 6 A-A"), corroborating the notion of a non-passive or non-diffusion mediated mechanism of transfer. In order to further support this view, transfer of DiD was comparatively tested in two different ways. On the one hand, by means of a DiD diffusion-preventing culture medium and by flow cytometric analysis. Cells were either cultured in normal medium (10% DMEM, 1% FCS, penicillin/streptomycin) or in normal medium supplemented with 1 % low melting point agarose, added 2 hours after plating (Bukoreshtliev et al., FEBS Lett. 2009, 6;583(9): 1481-8). Agarose-embedment of the cells did not have an influence on the average number of cells that received transfer (Fig. 2 C). Agarose treatment has been previously shown to stop diffusion of DiD stained cell debris. Further, agarose should inhibit any local medium currents that could be responsible for non-circular distribution of the transfer, and still the transfer was non-circular. Secondly, flow-cytometric measurements of cultures stained with CellTracker™Green CMFDA (5-chloromethylfluorescein diacetate; CTG) (Invitrogen Detection Technologies, Carlsbad, CA, USA) and DiD at a 1:1 ratio intensity of staining of the cells assessed with an Accuri Flow Cytometer. After gating out cell debris and doublets with the help of side scattered height discrimination, transfer was measured by the number of cells that contained DiD in CTG-stained cells. As a control, nascent CTG stained cells were used. These measurements showed that cell transfer increased with culture density up to a plateau, which was reached upon confluence (Fig. 7 A).

While the addition of low melting point agarose to DiD/CTG 1:1 ratio co-cultures considerably reduced the transfer to cells that were in direct contact with DiD stained cells, the extent of transfer reduction to cells without direct contact to DiD stained cells was even higher (Fig. 7 B-C'). This reduction strongly suggests that a substantial part of the cell transfer measured at DiD/CTG 1:1 ratio cultures by flow cytometric analysis occurs through a mechanism non-dependent on direct cell-to-cell contact, which could be diffusion or uptake of cell debris upon apoptosis. Since addition of agarose to the low ratio (1 :400) culture approach (Fig. 1) did not result in reduction of the transferred DiD signal, the transfer of DiD signal by diffusion does not seem to substantially interfere with these measurements (Fig. 2A, B). This reduction can be linked to lower number of donor cells present in the culture. By means of the present system, the transferred material can be allocated back to its cell of origin. Therefore, plating cells at low donor/acceptor ratio and imaging single donor cells surrounded in direct contact by a lawn of acceptor cells allows for more accurate measurement of contact dependent transfer as compared to stained/unstained 1:1 ratio cultures or flow cytometric approaches.

### g) BSA-coated stripes

To confirm contact-dependency of the DiD transfer, we plated DiD-donor and unstained-acceptor cells at a 1:100 ratio on a substrate that contained stripes coated with BSA (bovine serum albumin), which rendered this regions non-adhesive for the cells. Transfer did not occur across the non-adhesive stripes but frequently over distances exceeding the width of the stripe in other directions (Fig. 3A, B).

### h) Microfluidic chip

Furthermore, we plated two populations of HeLa Kyoto cells, DiD-stained and CTG-stained, onto two adjacent, non-overlapping areas with the help of microfluidics. Photolithography was performed in a MJB4 mask aligner (SUSS MicroTec AG, Garching, Germany) to fabricate SU-8 (MicroChem, MA, USA) patterned Si-masters for replica moulding in polydimethylsiloxane (PDMS). For the photolithography process, photo-emulsion or chromium masks were fabricated by GeSiM mbH (Grosserkmannsdorf, Germany) or Delta Mask B. V. (Enschede, The Netherlands) respectively based on the layout generated in LayoutEditor (Juspector UG, Unterhaching, Germany). The Si-master used for the casting of micro-fluidic chips contained a positive relief (100µm high) of a capillary network composed of three 300µm wide channels (inlets) that gradually converged into a single 900µm wide analysis channel (outlet). Si-masters used for micro-contact printing contained a battery of rectangular islands of size 100 x 40µm (L x W), 5µm deep and pitch between patterns of 20µm (short axis) and 60µm (long axis).

PDMS micro-fluidic chips were molded in a MicCell® casting station (GeSiM mbH) by pouring pre-mixed (10:1 ratio of base to curing agent) Sylgard 184 elastomer (Dow Corning, Ml, USA) onto the Si-master. The PDMS was cured at 65°C for 4 hours, cleaned with 3:2 isopropanol/ddH₂O (v/v) and placed into contact with a glutaraldehydeactivated glass coverslip (Mendelsohn et al., Langmuir. 2010, 15;26(12):9943-9) to seal the channels. After assembly, the capillary network was primed with 70% ethanol, washed with warm 0.01M PBS and incubated with 10µg/ml, fibronectin in PBS for 1 hour at 37°C. Chips were placed inside a humidified incubator overnight at 37°C prior to cell seeding.

PDMS stamps for micro-contact printing were casted and cleaned as described above. Afterwards, cut stamps were activated by exposure to oxygen plasma for 20 seconds and 0.2 mbar (Diener Electronic GmbH + Co. KG, Ebhausen, Germany). Activated stamps were inked with 50µg/mL fibronectin for 30 min at room temperature, briefly rinsed with PBS, followed by Milli-Q water (Millipore, MA, USA) and blown dry under a N2 stream. Inked stamps were then placed into contact with a glutaraldehydeactivated glass coverslip (Mendelsohn A.D. et al., Langmuir. 2010, 15;26(12):9943-9) for 1 min. Stamps were pealed-off and the coverslip was incubated for further 30 min at room temperature. The stamped coverslip was adhered to a 8-well flexiPERM® slide (Greiner Bio-one GmbH, Frickenhausen, Germany) and no-stamped areas were passivated with 2% (w/v) bovine serum albumin (Sigma-Aldrich) in PBS for 1 hour at room temperature.

### i) Cell culture and imaging within the microfluidic chip

Custom-made reservoirs were plugged into the inlets of the microfluidic chips. Reservoirs and the outlet of the microfluidic chip were connected to PID-controlled syringe pumps (Cetoni GmbH, Korbussen, Germany). The microfluidic set-up was then mounted in an Olympus IX70 microscope (Olympus Optical Co. Europe GmbH, Hamburg, Germany) equipped with a motorized XY stage and a piezo z-stepper (Prior Scientific Ins, MA, USA), a Polychrome V monochromator (T.I.L.L. Photonics, Gräfelfing, Germany) and a Andor DU-885 camera controlled by IQ 7.0 software (Andor Technology, Belfast, Northern Ireland). The imaging system was enclosed in an environmental chamber with controlled temperature (37°C), humidity and CO₂ (5%).

For cell seeding, pre-stained HeLa-Kyoto cells (2x10⁶ cells/ml) were suspended in DMEM supplemented with 1% foetal bovine serum (FBS) and injected in the reservoirs, while having a uniform flow rate of 3µl/min within the chip. CTG-stained cells were infused through contiguous inlets 1 and 2; DiD-stained counterparts were infused through inlet 3 to create a patterned co-culture of CTG and DiD-stained cells downstream of the inlet/outlet junction. After cell attachment, complete (normal) growth medium was infused at 30µl/min to remove unattached cells and then maintained at 10µl/min during 48 hours. Fluorescence images (16 bit) were acquired with a 40x / 1.40 NA oil-immersion objective at 10 random XY positions along the interface between CTG-and DiD-stained cells with an acquisition rate of 2 frames per hour.

The DiD-stained and CTG-stained cell populations as well as their interactions were imaged every hour over a period of 24 hours with wide-field fluorescence microscopy. It became clear that only those CTG-stained that had a direct contact with DiD-positive cells acquired DiD-stained organelles over time (Fig. 8 A). Furthermore, the DiD-stained organelles were located mainly at perinuclear positions, with vesicles shuttling in and out. The vesicles redistributed to the cell poles upon division (Fig. 8 A) and redistributed at the end of mitosis (not shown). Moreover, the perinuclear DiD accumulation seemed to maintain a dynamic interplay with DiD stained protrusion bundles originated from DiD-stained neighboring cells (Fig. 8 B).

The ultimate evidence for a contact-dependent transfer can be obtained by testing the co-culture of donor and acceptor cells in polydimethylsiloxane (PDMS) microfluidic chips, which facilitate the visualization of the contents of micro-channels or under the microscope (Mendelsohn A.D. et al., Langmuir. 2010, 15;26(12):9943-9). This approach was combined with time-lapse video microscopy, allowing real-time monitoring of cell-to-cell transfer. In this experiment, donor cells were stained with DiD and acceptor cells with CellTracker™Green (CTG) which enables imaging of living cells. By means of this set up, we could show that only acceptor cells (CTG) in direct-contact to donor (DiD) cells received DiD positive organelles within a period of 24 hours; see Fig. 8A. The mode of substance transfer of DiD-stained organelles was further linked to the formation of protrusion bundles originating from DiD positive donor cells as shown in Fig. 8B.

### j) Cell transfer dependency on F-actin filaments and serum

It has already been shown that TNT formation and cell-to-cell organelle transfer are dependent on F-actin (Gurke et al., Exp Cell Res. 2008, 10;314(20):366983). We set out to investigate whether F-actin and also serum play a role in the DiD-related specific transfer. In order to develop a method that allows deciphering the mechanism of contact-dependent cell-to-cell DiD transfer, we performed a co-culture with a DiD-stained/unstained 1:400 cell ratio. After 2 hours, the culture medium was exchanged to either normal culture medium containing Cytochalasin D or medium without serum. We fixed the cells after 18 hours, followed by staining with WGA-AF-488 (membrane) and Hoechst (nucleus). Subsequently, we imaged the cells by 3D confocal microscopy. To unambiguously quantify the amount of transferred material, we programmed a MatLab application that quantifies the integrated intensity of DiD signal inside the cells which were surrounding the DiD-stained donor cell. Either 100nM Cytochalasin D (Fig. 9B, C), an F-actin inhibitor, or serum starvation (Fig. 9 C) substantially inhibited DiD transfer as compared to control experiments (Fig. 9 A- C). We conclude that both F-actin and at least one serum component are required for contact-dependent cell-to-cell DiD transfer. In both cases, the transfer of DiD organelles was inhibited which suggests that the cell-to-cell substance transfer is an active process and requires confluency conditions (Fig. 9 C).

### k) Cell-to-cell contact sites

Since the requirement of F-actin and serum points toward an active process, we addressed the question whether DiD transfer occurred in compound package entities or rather diffusing via a membrane bridge. To get a visual impression of the structures that are involved in the transfer, we accomplished high resolution three dimensional and time-lapse acquisition imaging. Close examination of the contact sites (Fig. 10 A) disclosed structures protruding several micrometers under the surrounding cells, establishing a dense contact with the neighboring cells in intercalating fashion and, thereby, largely increasing the surface contact area in dense co-cultures. In less dense areas, CTG- and DiD-stained protrusions or protrusion bundles of varying diameter bridged the gaps between the cells (Fig. 10 A'). We observed both thin TNT-like structures hovering above the substrate as well as podosome-like structures stretching up to several cell diameters both along the ground between neighboring cells or on top of them. These structures mayor may not be different from the manifestations of the underlying machinery of the tunnelling nanotubes, which were previously found to support the transfer of DiD-stained organelles (Gurke et al., Exp Cell Res. 2008, 10;314(20):3669-83).

Moreover, we examined whether the cell-to-cell substance transfer (DiD) occurs in package entities or by diffusion via membrane bridges. Figure 10-B" show a *de novo* acquisition of DiD-organelles from a tip of a protrusions of a donor cell, which favors the notion of a compound transfer such as membrane-encased receptacles. Fig. 11A shows that the transferred DiD volume and the transferred integrated DiD intensity closely correlate. This result suggests a substance transfer by membrane-mediated enclosing of material as opposed to diffusion through a membrane bridge.

We could also observe *de novo* acquisition of DiD-organelles from the tip of protrusions of neighboring cells. This observation favors the notion of a compound transfer, probably membrane enclosed organelles, as opposed to diffusion through a membrane bridge. The fact that the transferred volume of DiD and the transferred integrated intensity almost perfectly correlate (Fig. 11A, B) contributed to the confirmation of transfer by membrane-mediated enclosing of material. We concluded that the volume of transferred DiD-signal varied, but not the average density of DiD within this compartment, therefore pointing toward the transport of fixed quanta of DiD rather than the diffusion of DiD through membrane connections. Of note, the measured transfer amount above the threshold of the greyscale value 100 (of 256) distributes around the median value of 145, while in background areas without DiD donor cell the measured signal value exhibit a median value of 160 (Fig. 11A, B), displaying a significantly different value of intensity per volume. In both cases, the values are well below the saturation mark of 256.

### Example 2- Contact-dependent transfer of Transferrin receptor

Several studies have shown the presence of organelles stained with fluorescent endocytosed lipid analogues and their subsequent transfer to connected cells, suggesting that endosomal sub-compartments may play a role in TNT-mediated organelles transfer (Gurke et al., Exp Cell Res. 2008 10;314(20):3669-83). We addressed this question by testing whether the transfer of the Transferrin receptor (Tf-R), a well-known marker of recycling endosomes, occurs between cell in low donor/acceptor ratio co-cultures at confluence. For this purpose, we transiently transfected the transferrin receptor fused to mCherry (Tf-RmCherry) in HeLa-Kyoto cells, which has been widely used to study intracellular trafficking of the transferrin receptor, and subsequently imaged.

### a) Plasmid mCherry-tagged Transferrin receptor and transfection

HeLa-Kyoto cells, provided by Dr. R. Pepperkok (EMBL, Heidelberg, Germany), were cultured in DMEM (GIBCO®, Invitrogen, Carlsbad, CA, USA) supplemented with 10% fetal calf serum (PAA Laboratories GmbH, Austria) at 37°C and 5% CO₂. When required, cells were detached from the culture dish by incubation in 0.05% Trypsin-EDTA (GIBCO®, Invitrogen, Carlsbad, CA, USA) at 37°C for 5 min, followed by a neutralization step with normal culture medium (DMEM plus 10% fetal calf serum).

A pJPA5 plasmid encoding for mCherry-tagged transferrin receptor (pJPA5-Tf-RmCherry) was obtained by cloning TfR cDNA into pmCherry purchased from Clontech Laboratories (AH Diagnostics, Oslo, Norway). pJPA5-Tf-RmCherry and pmCherry alone were transfected using Lipofectamine 2000 (Invitrogen, Carlsbad, CA, USA) following the manufacturer's instructions.

A population of donor cells consisting of Tf-RmCherry or mCherry alone expressing cells was co-cultured with an acceptor cell population, which were stained with CellTracker™Blue CMAC (CTB) (Invitrogen, Carlsbad, CA, USA), a non-transferable cytosolic dye, according to the manufacturer's instructions.

Unless otherwise stated, co-culture experiments were performed by seeding cells at a donor/acceptor 1:200 ratio in a confluent monolayer and further cultured for 20 hours in 2'-deoxythymidine.

For co-culture experiments, cell culture chambers (Ibidi u-chamber 8 wells, Inter Instrument AS, Norway) were coated for 1 hour at 37°C with 0.1 mg/mL poly-L- lysine (PLL) (Sigma-Aldrich Co., S1. Louis, MO, USA). Acceptor cells were stained with CellTracker™Blue for 45 minutes in complete medium at 37°C and washed two times for 30 minutes with culture medium at 37°C. Donor cells expressing mCherry construct were trypsinized, pelleted and co-cultured with acceptor cells at a donor/acceptor 1/10 ratio. Seeded cells were briefly centrifuged to accelerate attachment to the substrate and, 45 minutes later, cell culture medium was exchanged and replaced by medium supplemented with 2mM 2'-deoxythymidine (dThd) (Sigma- Aldrich Co., St. Louis, MO, USA) to inhibit cell division. 20 hours upon start of the co-culture, cells were either live-imaged or fixed using 4% paraformaldehyde/4% sucrose for 30 minutes, quenched with 50 m PBS NH₄Cl, and subsequently imaged.

### b) Microscopy and automated fluorescence quantification

Confocal microscopy was performed with a Leica TCS SP5 (Leica Microsystems GmbH, Wetzlar. Germany) and epifluorescent imaging using an Olympus IX70 microscope (Olympus Optical Co., Europe GmbH, Hamburg, Germany). For transfer experiments, we imaged fields of cells in co-cultures fulfilling the following criteria: (i) one donor object (either a single donor cell or a doublet abutted donor cells) (ii) surrounded by a confluent ring of acceptor cells and (iii) the entire field located at least at two cell diameter distance to the next closest donor cell.

Z-stacks of confocal planes were analyzed with or without their two first planes above the substrate level. To obtain cell segmentation of the donor cell, the mCherry channels were subjected to tophat filtering and Gaussian smoothing and cells were then detected from binary thresholding of the processed mCherry channel. The transfer channel (e.g. mCherry) was thresholded at value 100 to detect donor cells. The objects originated from the thresholding and with a volume large than a specified minimum cell volume were labeled as donor cell objects, and those connected to the image boundaries were removed. If no donor cells were detected, the image was labelled as control and four artificial donor cells were created at random positions. A binary transfer image was created from the mCherry channel by thresholding the mCherry channel at 130, wherein all signals inside the detected donor cells were removed, thus holding only transferred signals in the binary transfer image.

The volume for each donor cell was computed and a ring-region of 50 voxels distance (note: the voxel size was equal for all images) surrounding each donor cell object and corresponding to 22.5µm was created. The volume and total intensity of the transfer inside the ring was computed. This procedure was repeated for the ring intersected with the detected CellTracker™Blue (CBT) staining of acceptor cells. The latter resulted in measurements of local transfer inside cells, and the former, in measurements of total transfer localized around a donor cell object. The transfer estimations were repeated for the transfer channel. All transfer estimations were executed whilst the intensities at donor cell locations were removed. In order to restrict the quantification of transfer to the cytosol of acceptor cells and to avoid the observation of fluorescence outside the cells, the program quantified the fluorescence located in the CellTracker™Blue staining region of the ring and above the two first planes of the z- stack of images even if we measured no significant difference in the overall average of receptor transfer qualified on z-stacks with or without the two first planes (Fig. 13A and Fig. 14 B). For semi-automated transfer fluorescence quantification, a FIJI image analysis package was used, following essentially the same procedure as above. Before being summed, Z-stacks of images were treated in 2 dimensions and thresholded to 100 using control images. Regions corresponding to donor cell objects were detected automatically using the wand selection tool and a macro was run: a) to enlarge the 2pixel region, b) to compute integrated intensity and area, c) to cut, d) to enlarge by 48 pixel to create the ring-region and e) to compute its area and fluorescence intensity.

For the sake of clarity, we systematically present a maximal projection of z-stacks of confocal planes 2µm above the substrate to restrict the observation of fluorescence within the cytosol of acceptor cells. We also systematically present a z-axis re-slicing of regions that includes all the planes of the stacks.

Using this experimental set-up, we found that no transfer of mCherry alone was observed in CTB-stained acceptor cells (Fig. 12). However, a remarkable perinuclear signal for Tf-RmCherry within the cytosol of CTB-stained acceptor cells was detected upon co-culturing with Tf-RmCherry-expressing donor cells, indicating that the Transferrin receptor had indeed been transferred. Acceptor cells which were co-cultured with Tf-RmCherry-expressing cells but were not surrounded by donor cells, did not receive Tf-RmCherry (Fig. 12). Notably, the transfer was observed essentially in the first row of acceptor cells surrounding donor cells (Fig. 12 and Fig. 13A). Importantly, this transfer occurrence was confirmed in two additional cell lines, namely MDA-MB231 (provided by Prof. PE Lønning, Bergen) and osteosarcoma derived SAOS-2 cells (Prof. B.T. Gjertsen, Bergen), which were handled as the HeLa-Kyoto cell line (Fig. 15 and Fig. 16).

To automatically quantify the amount of transfer, we developed a program enabling the automated detection of donor objects (one or two cells abutted) and the quantification of the fluorescence intensities within them as well as within a 22.5µm region surrounding the donor cells (Fig. 13 A). For each donor object, the transfer was calculated as the fluorescence intensity of mCherry in the CTB-signal within the ring-region normalized to the fluorescence intensity of mCherry in the donor object. Despite the used of high-gain photomultiplier settings in order to detect weak mCherry signals in acceptor cells, mCherry signals collected in the red channel remained below the saturation level. This is shown by the linear correlation between the donor cell TfRmCherry fluorescence intensity and the donor object volume (Fig. 14 A).

By means of this method, we compared the amount of mCherry transfer to the CTB positive voxels within the ring region of donor cells expressing Tf-RmCherry or mCherry alone. As shown in Fig. 13B, the transfer of Tf-RmCherry was three-fold increased compared to transfer of MCherry alone, in any case remaining low. In order to determine the level of background fluorescence, areas of Tf-RmCherry/CTB co-cultures containing only acceptor cells and no donor cells were subjected to the same experimental procedures on simulated donor objects and ring-regions. We then calculated the average of fluorescence per ring-region as the fluorescence intensity of the ring normalized to the volume of the ring. Results show that the average fluorescence in the ring-region of control images was similar to the average fluorescence of mCherry cells, which. both did not represent more than 5% of the average fluorescence of Tf-RmCherry-expressing cells (Fig. 13 C). These observations show that, firstly, the transfer of the Transferrin receptor is independent of the presence of mCherry and, secondly, that it is not mediated by paracrine mechanisms through the culture medium. This conclusion is substantiated by the fact that acceptor cells cultured distantly from donor cells exhibited low rate of Tf-RmCherry.

### c) Contact-dependent transfer of Transferrin receptor involving TNTs

In order to reveal the mechanism underlying this transfer, we further co-cultured CTB-stained cells in medium conditioned by donor cells expressing TfRmCherry or mCherry alone in the same conditions as for the transfer experiment and compared to acceptor cells cultured in normal medium as control. Conditioned medium experiments were performed at the same as transfer experiments, thereby using the same cell populations, regions and culture conditions. In 8-well Ibidi u-chambers coated with PLL, 6000 donor cells and 25000 acceptor cells were plated in 300µL culture medium in separate wells. Cells were briefly centrifuged to accelerate cell attachment; donor cell culture medium was replaced by 2mM dthymidine and cells cultured for 20 hours. Conditioned media were collected and centrifuged for 10 minutes at 300g at 4°C to remove cell debris. Supernatants were added to a confluent layer of acceptor cells. After 20 hours in culture, wells were fixed and imaged by confocal microscopy.

The quantified fluorescence levels did not exceed background levels (Fig. 17A, B; t-test mCherry vs. control p=0.17; Tf-RmCherry vs. control p=0.07; TfRmCherry vs. mCherry p=0.58). These results therefore suggest that in our experimental conditions the transfer of the receptor is unlikely to involve micro-vesicles and exosome-mediated processes.

To further test whether the contact between cells may be required for the transfer of the receptor, we restricted the interactions between cells in co-cultures by seeding cells in micro-patterned surfaces with fibronectin stamped rectangular regions separated by 15µm. For this purpose, PDMS polymer stamps were prepared as follows: 14g base was added to 1.4g curing agent, mixed and casted on the master. After desiccation for 1 hour, PDMS was incubated 150 minutes at 70°C. PDMS stamps were then incubated for 30 minutes at room temperature with 15µL 50µg/mL fibronectin (Invitrogen, Carlsbad, CA, USA) in PBS, rinsed once with PBS, rinse again with water and dired before stamping on a petri dish coated with PLL. After removing the stamps, BSA 2% was added for 1 hour at room temperature to passivate non-stamped regions. Stamped-polymer petri dishes were washed with sterile PBS to avoid contamination. Cells were then seeded on the stamps and imaged using epifluorescence microscopy for 15 hours.

Live cell imaging of one donor cell expressing Tf-RmCherry in direct contact with two non-transfected cells and separated from three other non-transfected cells was performed immediately after seeding. Time-lapse imaging showed that cells physically separated from the donor cell by a distance of 15µm did not receive TfRmCherry, whereas cells in contact indeed received Tf-RmCherry (Fig. 18). Interestingly, thirty minutes before the receptor was detected in acceptor cells, the donor cell extended a Tf-RmCherry-positive bridge with the acceptor cell, suggesting that these protrusions may be involved in the process of transfer (Fig. 18).

Closer observation of the co-cultures with Tf-RmCherry-expressing cells and CTB-stained cells by confocal microscopy revealed that Tf-RmCherry-positive cellular protrusions extending towards acceptor cells seemed to be inserted within the CTB-signal structure as shown by orthogonal view along the z-axis of the z-stack images (Fig. 19 A). 3D reconstruction of these z-stacks indicated that cell-to-cell TfRmCherry positive bridges (Fig. 19 B) were inserted into the CTB-positive cytosol of acceptor cells. Furthermore, Tf-RmCherry-positive vesicles seemed to originate from the cell-to-cell bridges (Fig. 19 B).

Because Tf-RmCherry was present in cell-to-cell bridges we investigated whether the transfer took place as a result of plasma membrane uptake from the bridge or rather due to the transfer of intracellular organelles. We addressed this question by coupling Transferrin, the ligand of the Transferrin receptor, to AlexaFluor™546 red fluorescent fluorochrome (T23364, Invitrogen, Carlsbad, CA, USA). Transferrin is internalized together with the Transferrin receptor through the clathrin-mediated endocytic pathway and is known to allocate early and recycling endosomes within 5 and 30 minutes after internalization, respectively. We thus performed an internalization assay to test for the presence of Transferrin within tunnelling nanotubes (TNTs), which were identified by plasma membrane staining with wheat germ agglutinin as cell-to-cell bridges containing F-actin and suspending in the medium. 25.000 HeLa-Kyoto cells were seeded on covers lips coated with PLL and cultured in 2 mM d-Thymidine for 24 hours. Ice cold solution of culture medium without serum and containing Transferrin-AlexaFluor™546 (8µg/ml) was added to the cells on ice for 30 minutes. Cells were then rapidly washed on ice-cold PBS and fixed in 4% PFA for 30 minutes at 4°C or incubated at 37°C for 5 to 30 minutes, rapidly washed in ice-cold PBS and fixed in 4% PFA on ice. PFA was quenched with 50mM NH₄Cl PBS for 10 minutes and permeabilized for 30 seconds in 0.1 Triton™ X-100 (Sigma-Aldrich, St Louis, MO, USA). Cells were then stained with AlexaFluor™488-conjugated Phalloidin- (1/100) (A12379, Invitrogen, Carlsbad, CA, USA) and AlexaFluor™633-conjugated wheat germ agglutinin (invitrogen, Carlsbad, CA, USA) for 15 minutes at room temperature and imaged using confocal microscopy.

Whereas Transferrin was not observed in TNTs before internalization, Transferrin spots were detected in TNTs both 5 and 30 minutes after internalization, suggesting that both early and recycling endosomal-derived vesicles are present in TNTs (Fig. 20).

### Example 3 Manipulation of cell-to-cell transfer by siRNA knockdown

To investigate the transfer of DiD-stained organelles under gene knockdown, 24-well normal culture plates and 24-well plates with microscopy suitable bottom were coated with gelatine containing siRNAs and lipofectamine by the following procedure. 0.2% w/v Gelatine was dissolved in water and filtered (0.45µm pore size). 1.37g Sucrose was dissolved in OptiMEM. 6µl Sucrose/ OptiMEM solution was mixed with 1.75µl Lipofectamine™ 2000, 1.75µl water and 10µl silence-select siRNA (3µM), and this mix incubated for 20 minutes at room temperature together with 14.5µl gelatine solution. A 2-step 1:50 dilution in water was carried out, resulting in 1.6 ml volume. 300µL solution/well were dried in a vacuum centrifuged at 60°C, followed by a cooling step through application of vacuum at 37°C and stored in sealed boxes containing silica gel drying pearls as desiccant for up to several months. For experimental setup, HeLa-Kyoto cells were plated on siRNA coated plates before co-culture, either one day before co-culturing and at the assembly of the co-culture or just one day before co-culturing. In the latter case, 24-well plates with microscopy-suitable bottom were prepared as described above but without adding Lipofectamine 2000 and siRNA (see procedure described by Erfle et al in J Biomol Screen 2008 13: 575).

Since the transfer of DiD-positive material is contact-dependent, occurs in quanta and seems to be associated with membrane protrusions, we set out to further pinpoint its mechanism and, at the same, to develop a system to influence the cell-to-cell transfer, such that the cell-to-cell transport of proteins and/or pathogens can be influenced in a targeted specific fashion.

We screened for a list of educated guesses of genes coding for proteins which are probably involved in membrane trafficking (Fig. 21 A). We only continued to evaluate the candidate with the strongest scores of 2 to 3, which means that at least 2 of the 3 tested siRNAs directed against the same target reduced or increased the transfer by at least 20% with no contradicting tendency (the third siRNA against the same target having the same tendency as the other two siRNAs or being neutral). We also included candidate genes with only a moderate contradiction regarding the third construct. Importantly, the mean intensity/pixel did not deviate significantly from the median value of the control, even in the conditions treated with siRNAs that exhibited the strongest effect on the measured amount of transfer, between 37% and 147% (Fig. 11 B). In contrast, the mean intensity/pixel of DiD material found in control images with no donor cell within 200µm had, in comparison, a significantly higher value (Fig. 11 B). Therefore, while the chosen siRNAs seem to influence the number of packages transferred, intensity of DiD within the package is not affected.

In order to investigate the role of the knocked-down proteins in regard to their location in the donor or the acceptor cell, we chose a set of 20 of the candidates with the strongest effect on DiD transfer. For this purpose, we followed a protocol involving two transfections with the respective siRNA, two days prior analysis and one day prior analysis at the time point of co-culture assembly. We performed first a reference experiment, whereby only the first transfection step was carried out in which donor and acceptor cells were transfected. We found that some of the siRNAs which were transfected in both donor and acceptor cells reduced DiD transfer, which suggests either a function of the protein in both sides or a role of the protein in the transfer itself. In other cases, while knockdown only in donor cells resulted in a profound reduction of transfer, the knockdown in acceptor cells led to an increase in transfer. In contrast, transfection of certain siRNAs only showed a reduction in transfer when present in the acceptor cell and, an increase in transfer, if present in the donor cell.

These targeted siRNA-based knockdown experiments demonstrate that a manipulation of a contact-dependent transfer between cells and its quantifiable and automated monitoring is advantageous, enabling the investigation of, for example, pathogen transfer by large-scale screening of substances which may interfere with the transport of protein and viral entities.

### Example 4 - Manipulation of cell-to-cell transfer by exogenous expression of proteins

An alternative approach to influence the contact-dependent cell-to-cell transfer of material is the ectopic expression of mutant and wild type-tagged proteins. We transfected HeLa-Kyoto cell populations stably (by Lipofectamin 2000-mediated transfection), expressing control (of pEGFP-C1; Clontech Laboratories, Palo Alto, CA, USA) or EGFP-tagged candidate proteins, and subjected them to a low ratio co-culture (donor/acceptor 1:400) and measured the influence of the ectopically expressed proteins on cell-to-cell DiD transfer, and the transfer of the tagged proteins themselves.

### Example 5- Monitoring contact-dependent cell-to-cell transfer of HIV

For investigating the mechanism of virus transmission, a variety of methods are available for a labelling of viral particles. Viral particles can for example be labelled by chemically (covalent or non-covalent) or by fusion of fluorophores to proteins integrated into the viral particle. A genetic fusion of fluorophores to viral proteins further allows a permanent labelling of expressed target structures. When making use of the methods disclosed herein, a skilled person contemplates the following experiment.

First, a permanent labelling of HIV-virions for example by an amino-terminal fusion of GFP to Viral Protein R (Vpr) accessory protein for a visualization of the virus in living cells as described in McDonald et al., J Cell Biol. 2002, 159(3):441-52. This approach exploits the ability of Vpr to package into virions and to remain associated to the viral genome in the cytoplasm of infected cells. Labelled replication competent virus stocks can be achieved by cotransfection of HIV proviral DNA with GFP-HIV1-Vpr into 293T cells (Vodicka et al., Genes and Dev. 1998, 12:175-185) and purified (Dettenhoffer M. and Yu X.F., J.virol. 1999, 75:1460-1467). Exposure to stably CD4 transfected HeLa cells allows HIV infection (Kimpton & Emerman, J Virol 1992,66:2232- 2239). Subsequently the GFP signal can be detected as puncta throughout the cells (McDonald et al., J Cell Biol. 2002, 159(3):441-52). The co-localization of GFP fluorescence with protein of viral identity can further be confirmed by immunocytochemical analysis. Moreover, it is contemplated an incubation of infected cells with 10 µM DiD (Di1C₁₈ Molecular Probes) whether there is some co-localization of the labelled virus particles with DiD stained organelles (McDonald et al., J Cell Biol. 2002, 159(3):441-52). In any case, such type of donor cells can then be used for studying contact-dependent cell-to-cell transfer of HIV. In order to exclude the usual CD4 mediated transmission by infection, CD4 negative acceptor cells can be used.

Following the experimental set up described in Examples 1 and 2, the contact-dependent cell-to-cell transfer of DiD and/or GFP-positive HIV-virions between cells can be quantitatively analyzed and monitored. Thus, the disclosed method allows a differentiation of this type of contact-dependent spread from medium-mediated transfer/infection of HIV. The claimed method further allows a quantification of the number of cell-to-cell transferred viral particles with high accuracy and the cell-to-cell substance transfer can even be assigned to single cells from which viral transmission is initiated. The automated method further proves useful for a screening of viral-transport interfering substances so that the present method for quantitative analysis of contact-dependent cell-to-cell transfers contributes to the development of therapeutically valuable strategies with respect to a treatment of AIDS and other virally-mediated indications.

### Example 6 - Comparison of contact-devendent cell-to-cell transfer of different traceable transfer marker substance

Moreover, we tested the transfer efficiency of different transfer markers. Donor cells (Hela-Kyoto) expressing a fusion protein of the enhanced green fluorescence protein (EGFP) and myosin-X (Myo10) or a fusion protein of EGFP and myosin1a-heavy meromyosin or only EGFP as control have further been loaded and marked with a fluorescent lipophilic tracer (DiD) to examine contact-dependent transfer efficiency of different traceable substances. The results have been summarized in Figures 22A-D which show that both substances were transferred between cells. However, myosin-X, which is required in the formation of TNT, is transferred much more efficiently than DiD, which suggests that it can be used as a specific marker for contact-dependent transfers. The knockdown of myosin with siRNA inhibits contact-dependent transfers in general (Gousset et al, Cell Sci. 2013, 126(19):4424-35. doi: 10.1242/jcs.129239) and Fig. 21.

### Example 7- Monitoring contact-dependent cell-to-cell transfer of loaded vesicles (hypothetical example)

Exosomes are nanosized, secreted cell organelles that are released by a huge variety of different cell species, including mesenchymal stem cells (MSCs). Containing a combination of lipids and proteins as well as RNAs, exosomes participate in intercellular communication processes. Consequently, the person skilled in the art will appreciate that it may be useful to prepare nanosized non-secreted cell organelles that are not secreted but passed on via contact-dependent cell-to-cell substance transfer between cells to make drug targeting even more specific.

Due to the wide range of molecules such non-secreted nanosized cell organelles can enclose and because of their cell type specific assembly, these small membrane vesicles will get more and more in the focus of diagnosis, drug delivery and clinical applications. However, coupled with the fact that beneficial effects of MSC administration are controversially discussed, it appears very likely that not all MSC-derived nanosized organelle fractions will exert beneficial effects. To select for MSC organelles being able to efficiently suppress inflammation associated symptoms the method disclosed herein will allow a comparison of independent MSC-derived cell organelle preparations as to cell-targeting specificity and substance transfer efficiency.

A representative example for diseases which could be treated by the above identified method would be Graft-versus-Host Diseases (GvHD), cancer, and the large family of inflammatory diseases as well as acute inflammatory processes such as GvHD, stroke, heart-attack and neonatal damages of the brain and lungs.

## Claims

1. A microscope-based method for quantitative analysis of contact-dependent cell-to-cell molecular communication and transfer between cells, the method comprising the steps:
(i) obtaining of first singularized cells containing or expressing one or more traceable substances to provide donor cells;
(ii) obtaining of second singularized cells not containing or expressing said one or more traceable substances to provide acceptor cells;
(iii) combining said donor and acceptor cells in a ratio so that a majority of donor cells are surrounded by acceptor cells when grown in co-culture on a substrate and that the donor cells are essentially more than 200 micrometers apart from each other to obtain neighborhood regions wherein a contact-dependent cell-to-cell substance transfer can take place between said one donor cell and its surrounding acceptor cells,
(iv) culturing of said donor cells and acceptor cells in a culture medium in the presence of a substrate for a first period of time to allow donor and acceptor cells to attach to said substrate;
(v) replacing said culture medium by a medium wherein the proliferation of cells is inhibited and, optionally, washing cells attached to said substrate;
(vi) co-culturing of said donor and acceptor cells in said proliferation inhibiting medium for a second period of time to allow for contact-dependent cell-to-cell transfer between said donor and acceptor cells;
(vii) imaging of donor and acceptor cells to identify single donor cells which are more than 200 micrometers apart from the next donor cell but in contact to surrounding acceptor cells and thereby defining by microscopy a region of interest (ROI) containing in a space volume the identified single donor cell and a number of surrounding acceptor cells in contact relationship:
(viii) determining the amounts of traceable substance contained within the volume picture elements (volumetric pixel or voxel) of said region of interest (ROI) and defining the volume picture elements which are located within the cell boundaries of said donor and acceptor cells by cell segmentation; and
(ix) calculating the amount of traceable substance present within the volume elements located within the acceptor cell(s) surrounding the single donor cell to determine the amount of contact-dependent cell-to-cell substance transfer between said donor cell(s) and surrounding acceptor cell(s).

2. The method of claim 1, wherein in step (iii) said donor cells are combined with acceptor cells in a ratio ranging from 1:100 to 1:1,000,000.

3. The method of claim 1 or claim 2, comprising the use of a 3D cell culture in a matrix or host animal.

4. The method of claim 1 or claim 2, wherein images of consecutive microscopic planes are analyzed as to the intensity of traceable material over background in identified cell volumes.

5. The method of any claim 1 to 4, wherein identified substance transfers are further analyzed for vectorial properties.

6. The method of any claim 1 to 5, wherein said traceable substances are selected from one or more of the group consisting of dyes, fluorescence dyes, Dil, DiO, Lysotracker™, radioactive marker substances, luminescence dyes, fluorescence dyes, electroluminescing dyes, fluorescing proteins, luminescing proteins, fluorescing peptides, luminescing peptides, proteins coupled with a marker substance, peptides coupled with a marker substance, nanoparticles, quantum dots.

7. The method of any claim 1 to 6, wherein said traceable substances are selected from one or more biological materials of the group consisting of viruses, subviral agents, viroids, virusoids, satellite viruses, prions; lentiviruses, adenoviridae, herpesviridae, poxviridae, papovaviridae, parvoviridae, picornaviridae, annelloviridae, togaviridae, retroviridae, human immunodeficiency virus (HIV), rhabdoviridae, orthomyxoviridae, hepadnaviridae; labelled cell organelles, labelled cell vesicles, labelled artificial vesicles, bacteria or parasites.

8. The method as claimed in any claim 1 to 7, wherein said donor and acceptor cells are different cells types.

9. The method as claimed in any claim 1 to 8, wherein a test substance is present, removed or added to the culture medium.

10. The method as claimed in any claim 1 to 9, wherein the test substance is a pharmaceutical composition for affecting contact-dependent cell-to-cell transfer and transmission.

11. The method according to claim 10, wherein the transfer or transmission comprises an infectious or pathogenic material selected from group consisting of double stranded DNA, singe-stranded DNA, double-stranded RNA, (+)single-stranded RNA, (-)single-stranded RNA, HIV, prion proteins, bacteria or parasites.

12. Use of the method of any claim 1 to 11 for a screening of an agent effective against cell-contact dependent viral, bacterial and parasitic diseases.

13. Use of the method of any claim 1 to 11 for a screening of active compounds and agents for treatment of tumours, metabolism disorders, nervous system disorders, circulatory disorders.

14. Use of the method of any claim 1 to 11 for a screening of active compounds or agent for use in gene therapy, cell targeting and pharmacology.

## Patentansprüche

1. Mikroskopierverfahren für eine quantitative Analyse der kontaktabhängigen molekularen Kommunikation von Zelle-zu-Zelle und des Transfers zwischen Zellen, umfassend die Schritte:
(i) Gewinnen von ersten einzelnen Zellen, die eine oder mehrere nachverfolgbare Substanzen enthalten oder abgeben und damit Geberzellen sind;
(ii) Gewinnen von zweiten einzelnen Zellen, die die eine oder mehreren nachverfolgbaren Substanzen nicht enthalten oder abgeben und damit Empfängerzellen sind;
(iii) Zusammenbringen von Geberzellen und Empfängerzellen in einem Verhältnis, zu dem die meisten Geberzellen von Empfängerzellen umgeben sind, wenn sie in einer gemeinsamen Kultur auf einem Substrat vermehrt werden, und zwar so, dass die Geberzellen im Wesentlichen mehr als 200 Mikrometer voneinander entfernt sind, damit man Nachbarschaftsbereiche erhält, in denen ein kontaktabhängiger Substanztransfer von Zelle zu Zelle zwischen der einen Geberzelle und ihren umgebenden Empfängerzellen erfolgen kann;
(iv) Kultivieren der Geberzellen und der Empfängerzellen in einem Kulturmedium, wobei während einer ersten Zeitperiode ein Substrat vorhanden ist, das es den Geberzellen und den Empfängerzellen ermöglicht, sich auf dem Substrat festzusetzen;
(v) das Ersetzen des Kulturmediums durch ein Medium, in dem die Vermehrung der Zellen gehemmt wird, und wahlweise das Waschen der Zellen, die an dem Substrat haften;
(vi) in einer zweiten Zeitperiode das gemeinsame Kultivieren der Geberzellen und der Empfängerzellen in dem Medium, das die Zellvermehrung hemmt, damit ein kontaktabhängiger Transfer von Zelle zu Zelle zwischen den Geberzellen und den Empfängerzellen möglich ist;
(vii) das Abbilden der Geberzellen und der Empfängerzellen, damit einzelne Geberzellen gekennzeichnet werden, die mehr als 200 Mikrometer von der nächsten Geberzelle entfernt sind, jedoch Kontakt zu umgebenden Empfängerzellen haben, und mit Hilfe von Mikroskopie das Bestimmen eines interessierenden Bereichs (ROI), der in einem räumlichen Volumen die gekennzeichnete einzeine Geberzelle enthält sowie eine Anzahl umgebender Empfängerzellen, die dazu Kontakt haben;
(viii) das Feststellen der Mengen an nachverfolgbarer Substanz, die in den Volumenbildelementen (volumetrische Pixel bzw. Voxel) des interessierenden Bereichs (ROI) enthalten sind, und das Bestimmen der Volumenbildelemente, die sich innerhalb der Zellgrenzen der Geberzellen und Empfängerzellen befinden, durch Zellsegmentierung; und
(ix) das Berechnen der Menge an nachverfolgbarer Substanz, die in den Volumenelementen vorhanden ist, die sich in der Empfängerzelle bzw. den Empfängerzellen befinden, die die einzelne Geberzelle umgeben, damit der Umfang des kontaktabhängigen Substanztransfers von Zelle zu Zelle zwischen der Geberzelle bzw. den Geberzellen und der umgebenden Empfängerzelle bzw. den Empfängerzellen festgestellt wird.

2. Verfahren nach Anspruch 1, wobei im Schritt (iii) die Geberzellen mit Empfänger zellen in einem Verhältnis zusammengebracht werden, das zwischen 1:100 und 1:1000000 liegt

3. Verfahren nach Anspruch 1 oder 2, umfassend den Gebrauch einer 3D-Zellkultur in einer Matrix oder einem Wirtstier.

4. Verfahren nach Anspruch 1 oder 2, wobei Bilder von aufeinander folgenden mikroskopischen Ebenen auf die Intensität von nachverfolgbarem Material gegenüber einem Hintergrund in gekennzeichneten Zellvolumen untersucht werden.

5. Verfahren nach irgendeinem der Ansprüche 1 bis 4, wobei die gekennzeichneten Substanztransfers zudem auf vektorielle Eigenschaften untersucht werden.

6. Verfahren nach irgendeinem der Ansprüche 1 bis 5, wobei die nachverfolgbaren Substanzen aus einer oder mehreren Substanzen ausgewählt werden, die zu der Gruppe gehören, die umfasst: Farbstoffe, fluoreszierende Farbstoffe, Dil, DiO, Lysotracker™, radioaktive Markersubstanzen, Lumineszenzfarbstoffe, Fluoreszenzfarbstoffe, elektrolumineszierende Farbstoffe, fluoreszierende Proteine, lumineszierende Proteine, fluoreszierende Peptide, lumineszierende Peptide, Proteine, die mit einer Markersubstanz gekoppelt sind, Peptide, die mit einer Markersubstanz gekoppelt sind, Nanoteilchen und Quantenpunkte.

7. Verfahren nach irgendeinem der Ansprüche 1 bis 6, wobei die nachverfolgbaren Substanzen aus einem biologischen Material oder mehreren biologischen Materialien aus der Gruppe ausgewählt werden, die umfasst: Viren, subvirale Stoffe, Viroide, Virusoide, Satellitenviren, Prionen; Lentiviren, Adenoviridae, Herpesviridae, Poxviridae, Papovaviridae, Parvoviridae, Picornaviridae, Anelloviridae, Togaviridae, Retroviridae, menschliche Immunschwächeviren (HIV), Rhabdoviridae, Orthomyxoviridae, Hepadnaviridae, bezeichnete Zellorganellen, bezeichnete Zell-Vesikeln, bezeichnete künstlich hergestellte Vesikeln, Bakterien oder Parasiten.

8. Verfahren nach irgendeinem der Ansprüche 1 bis 7, wobei es sich bei den Geberzellen und den Empfängerzellen um unterschiedliche Zelltypen handelt.

9. Verfahren nach irgendeinem der Ansprüche 1 bis 8, wobei in dem Kulturmedium eine Testsubstanz vorhanden ist bzw. entfernt oder zugefügt wird.

10. Verfahren nach irgendeinem der Ansprüche 1 bis 9, wobei die Testsubstanz eine pharmazeutische Zusammensetzung ist, die den kontaktabhängigen Transfer von Zelle zu Zelle bzw. die Übertragung beeinflusst.

11. Verfahren nach Anspruch 10, wobei der Transfer bzw. die Übertragung ein infektiöses oder pathogenes Material umfasst, das aus der Gruppe ausgewählt wird, die umfasst: doppelsträngige DNA, einzelsträngige DNA, doppelsträngige RNA, (+)einzelsträngige RNA, (-)einzelsträngige RNA, HIV, Prionenproteine, Bakterien oder Parasiten.

12. Verwendung des Verfahrens nach irgendeinem der Ansprüche 1 bis 11 zum Screenen eines Mittels, das gegen zellkontaktabhängige virale, bakterielle oder von Parasiten verursachte Krankheiten wirksam ist.

13. Verwendung des Verfahrens nach irgendeinem der Ansprüche 1 bis 11 zum Screenen von aktiven Zusammensetzungen und Mitteln für die Behandlung von Tumoren, Stoffwechselstörungen, Störungen des Nervensystems und Störungen des Kreislaufs.

14. Verwendung des Verfahrens nach irgendeinem der Ansprüche 1 bis 11 zum Screenen von aktiven Zusammensetzungen und Mitteln für den Einsatz in der Gentherapie, dem Zelltargeting und der Pharmakologie.

## Revendications

1. Procédé par microscope pour l'analyse quantitative de communications et de transferts moléculaires intercellulaires dépendants du contact entre des cellules, le procédé comprenant les étapes suivantes :
(i) l'obtention de premières cellules singularisées contenant ou exprimant une ou plusieurs substances traçables pour fournir des cellules donneuses ;
(ii) l'obtention de secondes cellules singularisées ne contenant ni n'exprimant lesdites une ou plusieurs substances traçables pour fournir des cellules acceptrices ;
(iii) l'association desdites cellules donneuses et acceptrices selon un rapport de sorte qu'une majorité de cellules donneuses sont entourées de cellules acceptrices lorsqu'elles sont cultivées en culture conjointe sur un substrat et que les cellules donneuses se trouvent essentiellement à plus de 200 micromètres les unes des autres pour obtenir des régions de voisinage dans lesquelles un transfert de substance intercellulaire dépendant du contact peut se produire entre ladite cellule donneuse et ses cellules acceptrices environnantes,
(iv) la mise en culture desdites cellules donneuses et cellules acceptrices dans un milieu de culture en présence d'un substrat pendant une première période de temps pour permettre aux cellules donneuses et acceptrices de se fixer sur ledit substrat ;
(v) le remplacement dudit milieu de culture par un milieu dans lequel la prolifération des cellules est inhibée et, facultativement, le lavage des cellules fixées sur ledit substrat ;
(vi) la mise en culture conjointe desdites cellules donneuses et acceptrices dans ledit milieu inhibant la prolifération pendant une seconde période de temps pour permettre un transfert intercellulaire dépendant du contact entre lesdites cellules donneuses et acceptrices ;
(vii) la capture d'images de cellules donneuses et acceptrices pour identifier des cellules donneuses individuelles qui se trouvent à plus de 200 micromètres de la cellule donneuse suivante mais en contact avec des cellules acceptrices environnantes et identifier ainsi par microscopie une région d'intérêt (ROI) contenant dans un volume d'espace la cellule donneuse individuelle identifiée et un certain nombre de cellules acceptrices environnantes en relation de contact ;
(viii) la détermination des quantités de substance traçable contenues dans les éléments d'image de volume (pixel ou voxel volumétrique) de ladite région d'intérêt (ROI) et la définition des éléments d'image de volume qui se trouvent dans les limites de cellule desdites cellules donneuses et acceptrices par segmentation cellulaire ; et
(ix) le calcul de la quantité de substance traçable présente dans les éléments de volume situés dans la ou les cellules acceptrices entourant la cellule donneuse individuelle pour déterminer la quantité de transfert de substance intercellulaire et dépendant du contact entre la/lesdites cellules donneuses et la/lesdites cellules acceptrices environnantes.

2. Procédé selon la revendication 1, dans lequel à l'étape (iii) lesdites cellules donneuses sont associées à des cellules acceptrices selon un rapport allant de 1:100 à 1:1 000 000.

3. Procédé selon la revendication 1 ou la revendication 2, comprenant l'utilisation d'une culture cellulaire 3D dans une matrice ou un animal hôte.

4. Procédé selon la revendication 1 ou la revendication 2, dans lequel des images de plans microscopiques consécutifs sont analysées en termes d'intensité de la matière traçable sur un fond dans des volumes cellulaires identifiés.

5. Procédé selon l'une quelconque des revendications 1 à 4, dans lequel les transferts de substances identifiés sont analysés en outre pour leurs propriétés vectorielles.

6. Procédé selon l'une quelconque des revendications 1 à 5, dans lequel lesdites substances traçables sont choisies parmi un ou plusieurs éléments du groupe comprenant les colorants, les colorants de fluorescence, Dil, DiO, Lysotracker™, les substances marqueurs radioactifs, les colorants de luminescence, les colorants de fluorescence, les colorants électroluminescents, les protéines fluorescentes, les protéines luminescentes, les peptides fluorescents, les peptides luminescents, les protéines couplées avec une substance marqueur, les peptides couplés avec une substance marqueur, les nanoparticules, les points quantiques.

7. Procédé selon l'une quelconque des revendications 1 à 6, dans lequel lesdites substances traçables sont choisies parmi une ou plusieurs matières biologiques du groupe comprenant les virus, les agents subviraux, les viroïdes, les virusoïdes, les virus satellites, les prions; les lentivirus, les Adenoviridae, les Herpesviridae, les Poxviridae, les Papovaviridae, les Parvoviridae, les Picornaviridae, les Annelloviridae, les Togaviridae, les Retroviridae, le virus de l'immunodéficience humaine (VIH), les Rhabdoviridae, les Orthomyxoviridae, les Hepadnaviridae ; les organelles cellulaires marquées, les vésicules cellulaires marquées, les vésicules artificielles marquées, les bactéries ou les parasites.

8. Procédé selon l'une quelconque des revendications 1 à 7, dans lequel lesdites cellules donneuses et acceptrices sont des types cellulaires différents.

9. Procédé selon l'une quelconque des revendications 1 à 8, dans lequel une substance de test est présente dans le milieu de culture, éliminée dudit milieu ou ajoutée à celui-ci.

10. Procédé selon l'une quelconque des revendications 1 à 9, dans lequel la substance de test est une composition pharmaceutique destinée à affecter le transfert et la transmission intercellulaires dépendants du contact.

11. Procédé selon la revendication 10, dans lequel le transfert ou la transmission comprend une substance infectieuse ou pathogène choisie dans le groupe comprenant l'ADN double brin, l'ADN simple brin, l'ARN double brin, l'ARN simple brin(+), l'ARN simple brin(-), le VIH, les protéines prions, les bactéries ou les parasites.

12. Utilisation du procédé selon l'une quelconque des revendications 1 à 11 pour une sélection d'un agent efficace contre les maladies virales, bactériennes et parasitaires dépendantes d'un contact cellulaire.

13. Utilisation du procédé selon l'une quelconque des revendications 1 à 11 pour une sélection de composés et d'agents actifs pour le traitement des tumeurs, des troubles du métabolisme, des troubles du système nerveux et des troubles circulatoires.

14. Utilisation du procédé selon l'une quelconque des revendications 1 à 11 pour une sélection de composés ou d'agents actifs destinés à être utilisés en thérapie génique, en ciblage cellulaire et en pharmacologie.
